(19) Europäisches Patentamt
European Patent Office
Office européen des brevets

(11) Veröffentlichungsnummer: **0 137 442**
**A2**

(12) # EUROPÄISCHE PATENTANMELDUNG

(21) Anmeldenummer: 84111746.8

(22) Anmeldetag: 02.10.84

(51) Int. Cl.⁴: C 07 D 501/46
A 61 K 31/545

(30) Priorität: 08.10.83 DE 3336755
22.10.83 DE 3338440

(43) Veröffentlichungstag der Anmeldung: 17.04.85 Patentblatt 85/16

(84) Benannte Vertragsstaaten: AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Lattrell, Rudolf, Dr.
Heuhohlweg 6h
D-6240 Königstein/Taunus(DE)

(72) Erfinder: Blumbach, Jürgen, Dr.
Manderscheider Strasse 13b
D-6000 Frankfurt am Main 71(DE)

(72) Erfinder: Dürckheimer, Walter, Dr.
Im Lerchenfeld 45
D-6234 Hattersheim am Main(DE)

(72) Erfinder: Schwab, Wilfried, Dr.
Am Flachsland 18
D-6233 Kelkheim (Taunus)(DE)

(72) Erfinder: Seeger, Karl, Dr.
Schwalbenweg 9
D-6238 Hofheim am Taunus(DE)

(54) **Cephalosporinderivate und Verfahren zu ihrer Herstellung.**

(57) Cephemderivate der allgemeinen Formel

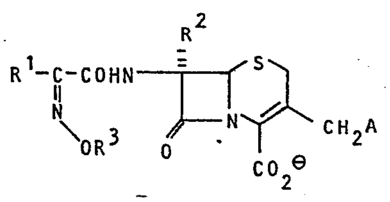

und deren physiologisch verträgliche Säureadditionssalze, gegen bakterielle Infektionen wirksame pharmazeutische Präparate, die solche Cephemderivate enthalten, Verfahren zur Herstellung der Cephemderivate und der pharmazeutischen Präparate, sowie die Verwendung der Cephemderivate zur Bekämpfung bakterieller Infektionen.

EP 0 137 442 A2

Croydon Printing Company Ltd.

Cephalosporinderivate und Verfahren zu ihrer Herstellung

Die Erfindung betrifft neue Cephalosporinderivate und Verfahren zu ihrer Herstellung, insbesondere polare Cephemderivate, die in 3'-Stellung des Cephemrings durch eine quaternäre Ammoniogruppe substituiert sind und die eine sehr gute antimikrobielle Wirkung gegen gram-positive und gram-negative Bakterien besitzen und deshalb als Arzneimittel zur Behandlung von mikrobiellen Infektionen geeignet sind.

Gegenstand der Erfindung sind daher Cephemderivate der allgemeinen Formel I

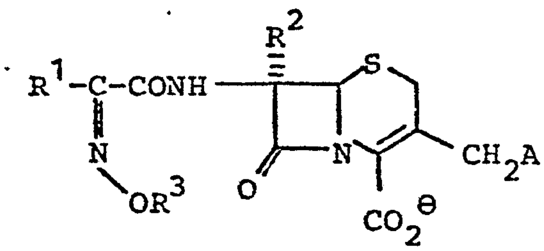

(I)

und deren physiologisch verträgliche Säureadditionssalze worin

$R^1$ einen 5- oder 6-gliedrigen heterocyclischen Ring bedeutet, der durch folgende Formeln wiedergegeben wird:

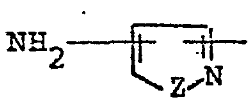 , 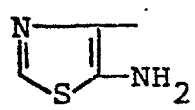 , 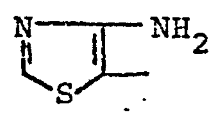 ,

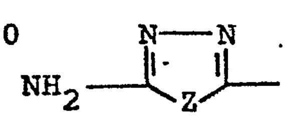 , 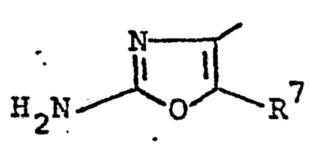 ,

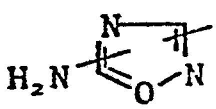 , 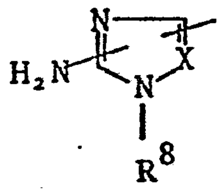 , 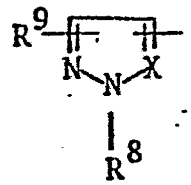 , 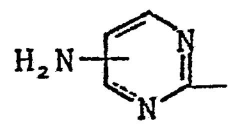 ,

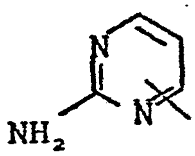 , 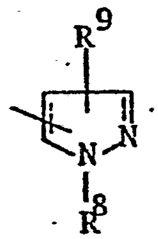 , 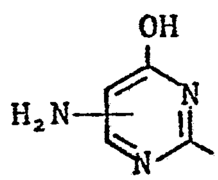 , 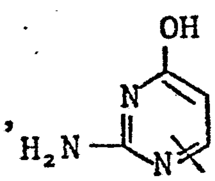 ,

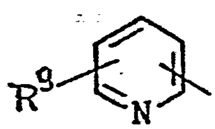 , 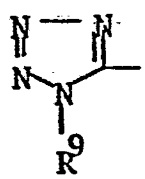 , 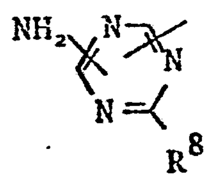 , 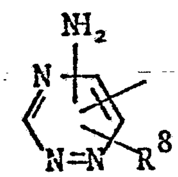 ,

worin $R^7$ für Wasserstoff oder Halogen, $R^8$ für Wasserstoff oder einen gegebenenfalls substituierten $C_1$-$C_4$-Alkylrest, $R^9$ für Wasserstoff oder Amino, X für N oder CH, Z für O,S oder $NR^8$ steht,

$R^2$ Wasserstoff oder Methoxy

$R^3$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $-(CH_2)_n-\overset{R^4}{\underset{R^5}{(\overset{|}{\underset{|}{C}})_m}}-R^6$, worin m und n jeweils für 0 oder 1 steht

und

$R^4$ und $R^5$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$Alkyl- und die $C_3$-$C_7$-Cycloalkylidengruppe noch weiter ein- oder mehrfach substituiert sein können,

$R^6$ eine COOH-; CN- oder $CONH_2$-Gruppe,

A eine gegebenenfalls substituierte Ammoniogruppe, die von einem tertiären aliphatischen oder cyclischen Amin abgeleitet ist;

ein ungesättigtes, gegebenenfalls substituiertes 5-6-gliedriges heterocyclisches Kation der Formel 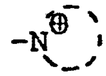 , das weitere N, O oder S-Atome im Ring enthalten kann, beispielsweise Triazolio, Imidazolio, Pyrimidinio, Pyrazolio, Pyridazinio, Pyrazinio, Thiazolio, Isothiazolio, Pyrrolio, Oxazolio, und an das zusätzliche Ringe ankondensiert sein können z.B. azinio, Chinazolinio, Chinoxalinio, Cinnolinio;

einen Chinolinium 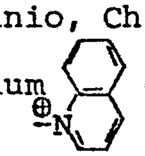 oder einen Isochinoliniumrest 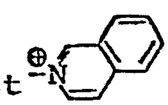, die jeweils auch ein- oder mehrfach, gleich oder verschieden substituiert sein können durch gegebenfalls substituiertes $C_1$-$C_6$-Alkyl, $C_1$-$C_6$-Alkoxy, Halogen, Trifluormethyl oder Hydroxy,

oder einen Phenanthridiniumrest,

oder einen Pyridiniumrest 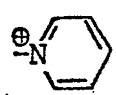, der ein- oder mehrfach, gleich oder verschieden substituiert sein kann durch gegebenfalls substituiertes $C_1$-$C_6$-Alkyl, wobei 2 orthoständige Alkylgruppen auch zu einem gegebenfalls substituierten Di- bis Decamethylen-Ring geschlossen sein können, in dem ein Ring-C-Atom durch ein Heteroatom ersetzt sein kann und weiterhin auch noch eine oder zwei Doppelbindungen enthalten sein können; durch gegebenfalls substituiertes $C_2$-$C_6$-Alkenyl, durch $C_2$-$C_6$-Alkinyl, durch $C_3$-$C_7$-Cycloalkyl oder $C_3$-$C_7$-Cycloalkylmethyl, durch $C_4$-$C_7$-Cycloalkenyl, durch gegebenenfalls

substituiertes $C_1$-$C_6$-Alkyloxy, durch $C_3$-$C_7$-Alkinyloxy, durch $C_2$-$C_6$-Alkenyloxy, durch Halogen, Trifluormethyl oder Hydroxy, durch gegebenenfalls substituiertes Phenyl oder Benzyl, durch Formyl oder ketalisiertes Formyl, durch gegebenenfalls substituiertes $C_1$-$C_6$-Alkylcarbonyl, das auch in ketalisierter Form vorliegen kann; durch Arylcarbonyl oder Carbamoyl, bedeutet und in der die $R^3$ O-Gruppe in syn-Position steht.

Die vorliegende Erfindung ist insbesondere auf Verbindungen gerichtet, in denen $R^1$, $R^2$, $R^7$, $R^8$, $R^9$, X und Z die vorstehenden Bedeutungen besitzen und

<u>$R^3$</u> = Wasserstoff, $C_1$-$C_6$-Alkyl, das ein- oder mehrfach substituiert sein kann durch Halogen, $C_1$-$C_6$-Alkylthio, $C_1$-$C_6$-Alkoxy, Aryl oder Heteroaryl, $C_2$-$C_6$-Alkenyl, das ein- oder mehrfach substituiert sein kann durch Halogen; $C_2$-$C_3$-Alkinyl, $C_3$-$C_6$-Cycloalkyl, $C_3$-$C_6$-Cycloalkyl-$C_1$-$C_6$-alkyl, $C_4$-$C_7$-Cycloalkenyl und in der die Gruppe

$(CH_2)_n(\overset{R^4}{\underset{R^5}{C}})_mR^6$ die vorstehende Bedeutung hat;

<u>A</u> eine Ammoniogruppe, die von einem tertiären aliphatischen oder cyclischen Amin abgeleitet ist, das durch $C_1$-$C_4$-Alkyl, Hydroxy, $C_1$-$C_4$-Alkyloxy, Oxo, Halogen, Di-$C_1$-$C_4$-Alkylamino, Trifluormethyl, Sulfo, gegebenenfalls derivatisiertes Carboxy, Cyano, substituiert sein kann; ein heterocyclisches Kation der Formel $-\overset{\oplus}{N}$ ), wie z.B. ein 1,2,3- oder 1,2,4-Triazolio, die durch $C_1$-$C_4$-Alkyl substituiert sein können; ein gegebenenfalls mit $C_1$-$C_4$-Alkyl substituiertes Imidazolio oder Pyrazolio; ein Pyrimidinio, das gegebenenfalls mit Amino oder Halogen substituiert ist; ein Pyridazinio oder Pyrazinio, die durch Halogen oder durch $C_1$-$C_4$-Alkyl substituiert sein können und wobei zwei orthoständige Alkylgruppen auch zu einem gegebenenfalls substituierten Di-bis Heptamethylenring geschlossen sein können; ein Thiazolio- oder Oxazolio, die durch Hydroxy-$C_1$-$C_4$-Alkyl und/oder $C_1$-$C_4$-Alkyl substituiert sein können;

einen Chinolinium- oder einen Isochinoliniumrest, die jeweils auch ein- oder mehrfach, gleich oder verschieden substituiert sein können durch $C_1$-$C_6$-Alkyl, das substituiert sein kann durch Hydroxy; durch $C_1$-$C_6$-Alkoxy, durch Halogen, durch Trifluormethyl, durch Hydroxy oder

einen Pyridiniumrest 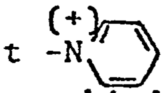 bedeutet, der ein- oder mehrfach, gleich oder verschieden substituiert sein kann durch $C_1$-$C_6$-Alkyl, das ein- oder mehrfach substituiert sein kann durch Hydroxy; Formyl oder $C_1$-$C_6$-Alkylcarbonyl, deren Carbonylgruppen auch in ketalisierter Form vorliegen können; Sulfo, Carbamoyl, $C_1$-$C_6$-Alkyloxy, Hydroxy-$C_1$-$C_6$-alkyloxy und wobei 2 Alkylgruppen auch zu einem gegebenenfalls substituierten Di- bis Decamethylen-ring geschlossen sein können, in dem ein Ring-C-Atom durch ein Heteroatom ersetzt sein kann und weiterhin auch noch eine oder zwei Doppelbindungen enthalten sein können; durch $C_2$-$C_6$-Alkenyl, das durch Hydroxy substituiert sein kann; durch $C_3$-$C_6$-Alkinyl, durch $C_3$-$C_7$-Cycloalkyl oder $C_3$-$C_7$-Cycloalkylmethyl; durch $C_4$-$C_7$-Cycloalkenyl; durch $C_1$-$C_6$-Alkoxy, das durch Hydroxy substituiert sein kann; durch $C_2$-$C_6$-Alkenyloxy oder $C_3$-$C_6$-Alkinyloxy; durch Halogen, Trifluormethyl oder Hydroxy; durch Phenyl oder Benzyl, die auch durch Halogen substituiert sein können; durch Formyl, oder ketalisiertes Formyl; durch $C_1$-$C_6$-Alkylcarbonyl, das auch durch Hydroxy substituiert sein und auch in ketalisierter Form vorliegen kann; durch Arylcarbonyl oder Carbamoyl und wobei auch bei diesen bevorzugten, unter die allgemeine Formel I fallenden Verbindungen die $R^3O$-Gruppe in syn-Position steht.

Als gegebenenfalls mögliche Substituenten des unter A erwähnten Di- bis Decamethylen-Rings, in dem ein Ring-C-Atom durch ein Heteroatom ersetzt sein kann und weiterhin auch noch eine oder zwei Doppelbindungen enthalten

sein können, kommen insbesondere die folgenden Substituenten in Betracht, die ein- oder mehrfach, vorzugsweise jedoch einfach auftreten können:

$C_1$-$C_6$-Alkyl, $C_1$-$C_4$-Alkoxy, Hydroxymethyl, Halogen, Hydroxy, Oxo, Exomethylen.

Der an den Pyridiniumrest ankondensierte Ring kann 2 bis 10 Ringglieder (Di- bis Decamethylen), vorzugsweise jedoch 3 bis 5 Ringglieder enthalten und somit beispielsweise einen Cyclopenteno-, Cyclohexeno- oder Cyclohepteno-Ring darstellen. Enthält ein solcher ankondensierter Ring eine Doppelbindung, so seien als Beispiele ein Dehydrocyclopentadieno-, Dehydrocyclohexadieno- oder Dehydrocycloheptadieno-Ring genannt. Ist in derartigen Ringen ein C-Atom durch ein Heteroatom ersetzt, so kommen als Heteroatome insbesondere Sauerstoff oder Schwefel in Betracht. Als ein Sauerstoffatom enthaltende, ankondensierte Ringe, die zwei oder eine Doppelbindung enthalten, seien beispielsweise erwähnt Furo, Pyrano, Dihydrofuro und Dihydropyrano; als ankondensierte Ringe mit einem Schwefelatom, die zwei oder eine Doppelbindung enthalten Thieno, Thiopyrano, Dihydrothieno und Dihydrothiopyrano. Von den ein Heteroatom enthaltenden, ankondensierten Ringen kommen für eine Substitution, insbesondere durch die vorstehend angegebenen Substituenten, insbesondere diejenigen Ringe in Betracht, die nur eine Doppelbindung enthalten.

Als besonders bevorzugt kommen beispielsweise die folgenden Substiuenten in Betracht:

$-R^1-$:

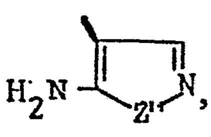, 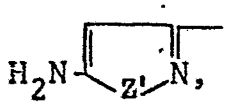, 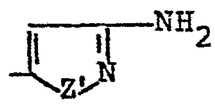 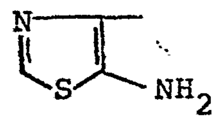 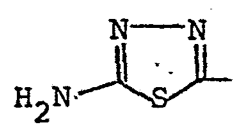

mit Z' = O, S

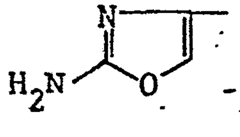 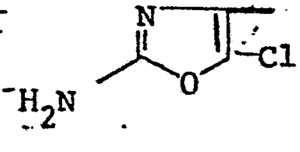 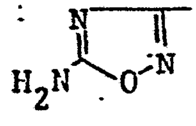 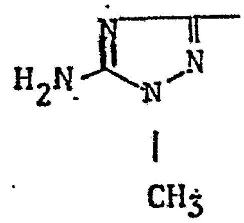

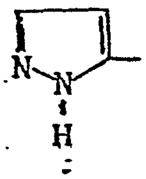
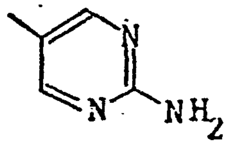
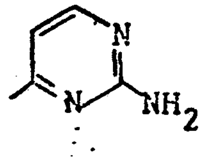
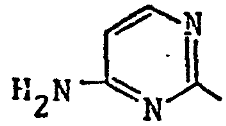

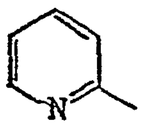
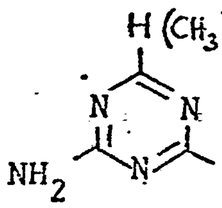
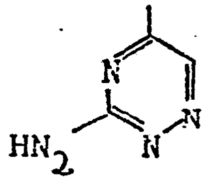
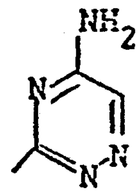

$R^2$: Wasserstoff oder $OCH_3$;

$R^3$: Wasserstoff, $C_1$-$C_6$-Alkyl, wie z.B. Methyl, Ethyl, Propyl, Isopropyl, Butyl, vorzugsweise Methyl, Ethyl; $C_1$-$C_2$-Halogenalkyl, z.B. Chlor, Brom, Jod oder Fluorsubstituiertes Alkyl, vorzugsweise Trifluorethyl, Difluormethyl, 2,2,3,3,-Tetrafluorpropyl ; durch $C_1$-$C_2$-Alkyloxy-$C_1$-$C_2$-alkyl, z.B. Äthoxyäthyl; $C_1$-$C_2$-Alkylmercapto-$C_1$-$C_2$-alkyl, z.B. Methylmercaptomethyl; durch Aryl, wie z.B. Phenyl, Tolyl, Chlorphenyl, substituiertes Alkyl, insbesonders Benzyl;
durch Heteroaryl substituiertes Alkyl, wie z.B. 1,3-Thiazol-4-yl-substituiertes Alkyl, insbesondere 1,3-Thiazol-4-yl-methyl,
$C_2$-$C_6$-Alkenyl, wie z.B. Vinyl, Allyl, Isopropenyl, Methallyl, insbesondere Allyl, Methallyl;
durch Halogen, wie z.B. durch Chlor oder Brom substituiertes $C_2$-$C_6$-Alkenyl, insbesondere 3-Chlor-propen-2-yl, 2-Brom-propen-2-yl, 2-Chlorpropen-2-yl;
$C_2$-$C_3$-Alkinyl, wie insbesondere Propargyl;
$C_3$-$C_7$-Cycloalkyl, wie insbesondere Cyclopropyl, Cyclobutyl, Cyclopentyl;
$C_3$-$C_7$-Cycloalkyl-$C_1$-$C_6$-alkyl, wie insbesondere Cyclopropylmethyl, Cyclobutylmethyl;
$C_4$-$C_7$-Cycloalkenyl, wie insbesondere Cyclopenten-1-yl;

die Gruppe $(CH_2)_n-(\overset{R^4}{\underset{R^5}{C}})_m-R^6$, wobei $R^6$ die Gruppe COOH, CN oder $CONH_2$ bedeutet und $R^4$ und $R^5$ gleich oder ver-verschieden sein können und Wasserstoff, Aryl, vorzugsweise Phenyl; $C_1$-$C_4$-Alkyl, wie z.B. Methyl, Ethyl, Propyl, Isoproyl, Butyl, sec. Butyl, vorzugsweise Methyl, Ethyl, insbesondere Methyl, bedeuten können oder

wobei $R^4$ und $R^5$ zusammen mit dem Kohlenstoffatom, an das sie gebunden sind, eine Methylengruppe oder eine $C_3$-$C_7$-Cycloalkylidengruppe bilden können, wie z.B. Cyclopropyl, Cyclobutyl, Cyclopentyl, Cyclohexyl, Cycloheptyl, vorzugsweise Cyclopropyl, Cyclobutyl, Cyclopentyl oder Cyclohexyl, und wobei die Cycloalkylidengruppe substituiert sein kann, z.B. durch $C_1$-$C_4$-Alkyl, vorzugsweise Methyl, oder durch Halogen, vorzugsweise Fluor und Chlor,

$m$ = O oder 1

$n$ = O oder 1, wobei die Summe von m und n 1oder 2 darstellt.

Bevorzugte Beispiele für die Gruppe- $(CH_2)_n(\overset{R^4}{\underset{R^5}{C}})_m$- sind die folgenden:

Für den Fall, daß n = O und m = 1 ist;

$-CH(CH_3)$, $-C(CH_3)_2$, $-CH(C_6H_5)$,

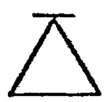 , 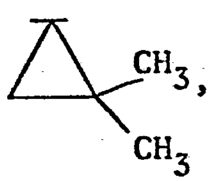 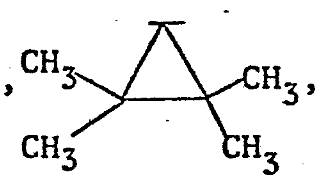 , 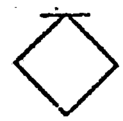 ,

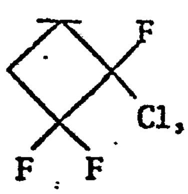 , 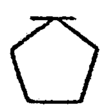 , 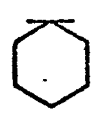 ,

für den Fall, daß m = O und n = 1 ist: $-CH_2-$ und falls n und m = 1 sind: $-CH_2-C(=CH_2)-$.

A: Ammonio, das von tertiären organischen aliphatischen oder cyclischen Aminen abgeleitet ist, beispielsweise $C_1$-$C_4$-Alkylammonio, wie insbesondere Trimethyl, Triäthyl-, Tripropyl-, Dimethyläthyl-, Dimethylbenzyl-, Dimethylpropargylammonio; $C_4$-$C_7$-Cycloalkylammonio, wie insbesondere 1-Methylpiperidinio, 1-Methylmorpholinio, 1,4-Dimethylpiperazinio und wobei die Ammoniogruppe ein- oder mehrfach, vorzugsweise 1- bis 3-fach substituiert sein kann, beispielsweise durch

$C_1$-$C_4$-Alkyl, wie insbesondere Methyl, Äthyl;
Hydroxy, wie z.B. in 1-(ß-Hydroxyäthyl)-morpholinio, 4-Hydroxy-1-methylpiperidinio, 1-Äthyl-3-hydroxypyrrolidinio;
$C_1$-$C_4$-Alkyloxy, insbesondere Methyloxy, Äthyloxy;
Oxo, wie z.B. in 1-Methyl-4-oxo-piperidinio;
Halogen, insbesondere Chlor, wie z.B. in 4-Chlor-1-methylpiperidinio;
Di-$C_1$-$C_4$-Alkylamino, wie insbesondere Dimethylamino;
Trifluormethyl, Sulfo, Sulfoniederalkyl, z.B. Sulfomethyl;
Carboxy, $C_1$-$C_4$-Alkyloxycarbonyl, wie insbesondere Methoxy-Äthoxycarbonyl; Carbamoyl, Cyano;

ein heterocyclisches Kation der Formel 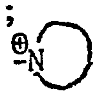, wie z.B. 3-Methyl-1-(1,2,3-triazolio), 2-Methyl-1-(1,2,3-triazolio), 4-Methyl-1-(1,2,4 triazolio), 3-Methyl-1-(1,3,4-triazolio), 3-Methyl-1-imidazolio, 2-Amino-1-pyrimidinio, 3-Methyl-1-pyrimidinio, 2-Hydroxyäthyl-1-pyrazolio, 2-Methyl-1-pyrazolio, Pyridazinio, 3-Methyl-1-pyridazinio, Isothiazolio, 2,5-Dimethyl-1-pyrazinio, Pyrazinio, Thiazolio, 4-Methylthiazolio, Phthalazinio, 5,6,7,8-Tetrahydrophthalazinio, 3,4-Trimethylenpyridazinio, 4,5-Trimethylenpyridazinio, Cinnolinio, 5,6,7,8-Tetrahydrocinnolinio, Chinazolinio, 5,6,7,8-Tetrahydrochinazolinio, Chinoxalinio, 5,6,7,8-Tetrahydrochinoxalinio;

einen Chinolinium- oder einen Isochinoliniumrest, die jeweils auch ein- oder mehrfach, gleich oder verschieden substituiert sein können durch:

$C_1$-$C_6$-Alkyl, wie z.B. Methyl, Ethyl, Propyl, Isopropyl, vorzugsweise Methyl; durch Methoxy

0137442

durch Hydroxy, durch Halogen oder Trifluormethyl;

einen <u>Pyridiniumrest</u>, der ein- oder mehrfach, vorzugsweise 1- bis 3-fach, insbesondere 1- bis 2-fach substituiert sein kann, beispielsweise durch $C_1$-$C_4$-Alkyl, wie insbesondere Methyl, Ethyl, Propyl, Isopropyl, n-Butyl, sec.-Butyl, tert.-Butyl, Dimethyl, Trimethyl, Methyl und Ethyl, Methyl und Propyl, Methyl und Isopropyl, Ethyl und Ethyl;
Hydroxy-$C_1$-$C_4$-alkyl, wie insbesondere Hydroxymethyl, Hydroxyethyl, Hydroxypropyl, Hydroxyisopropyl, Hydroxybutyl, Hydroxy-sec.-butyl oder Hydroxy-tert.-butyl, und wobei z.B. auch zwei oder drei Hydroxygruppen am Alkylrest stehen können; Formyl-$C_1$-$C_4$-alkyl, wie insbesondere Formylmethyl, $C_1$-$C_4$-Alkylcarbonyl-$C_1$-$C_4$-alkyl, wie insbesondere Methylcarbonylmethyl, Ethylcarbonylmethyl, Methylcarbonylethyl und Ethylcarbonylethyl; Sulfo-$C_1$-$C_4$-alkyl, wie 4-Sulfoäthyl; $C_3$-$C_4$-Alkenyl, wie insbesondere Allyl, 2-Methylallyl und Buten-3-yl, die auch noch durch Hydroxy substituiert sein können, wie insbesondere Hydroxyallyl und Hydroxybutenyl; $C_3$-$C_6$-Alkinyl, wie insbesondere Propargyl; $C_3$-$C_6$-Cycloalkyl und und $C_3$-$C_6$-Cycloalkyl-methyl, wobei sich die Kohlenstoffzahl auf den Cycloalkylteil bezieht, wie insbesondere Cyclopropyl, Cyclobutyl, Cyclopentyl und Cyclopentylmethyl;
$C_4$-$C_7$-Cycloalkenyl, wie insbesondere Cyclopenten-1-yl und Cyclohexen-1-yl;
$C_1$-$C_6$-Alkoxy, wie insbesondere Methyl- und Ethyloxy; Halogen, wie insbesondere 3-Fluor, 3-Chlor, 3-Brom, 3-Jod; Hydroxy, insbesondere 3-Hydroxy; Trifluormethyl, insbesondere 3-Trifluormethyl; Phenyl und Benzyl, die auch substituiert sein können, beispielsweise durch Halogen, insbesondere Chlor, wie z.B. 4-Chlorbenzyl; 2'-Thienyl und 3'-Thienyl;

$C_1$-$C_4$-Alkylcarbonyl; insbesondere Acetyl und Propionyl, vorzugsweise Acetyl; Formyl, Benzoyl, Carbamoyl.

Stellt A einen Pyridiniumrest dar, der durch zwei zu einem Di- bis Decamethylen-Ring geschlossene Alkylgruppen substituiert ist, der wiederum ein- oder mehrfach, vorzugsweise einfach substituiert sein und eine oder zwei Doppelbindungen enthalten kann, so kommen hierfür ganz besonders die folgenden ankondensierten Ringsysteme in Betracht:

Cyclobuteno, Cyclopenteno, Hydroxycyclopenteno, Oxocyclopenteno, Hydroxymethylcyclopenteno, Exomethylencyclopenteno, Carboxycyclopenteno und Carbamoyl-cyclopenteno,
Cyclohexeno, Hydroxycyclohexeno, Oxocyclohexeno, Hydroxymethyl-cyclohexeno, Exomethylen-cyclohexeno, Carboxycyclohexeno und Carbamocyclohexeno,

Cyclohepteno, Hydroxy-, Oxo-, Hydroxymethyl-, Exomethylen-, Carboxy-cyclohepteno und Carbamoyl-cyclohepteno; Dehydrocyclopenteno, Dehydro-cyclohexeno und Dehydro-cyclohepteno.

Ist in den vorstehend genannten ankondensierten Ringsystemen ein Ring-C-Atom durch ein Heteroatom, insbesondere Sauerstoff ersetzt, so kommen insbesondere in Betracht: Furo(2,3-b)pyridin, Furo (3,2-b) pyridin, Furo(2,3-c) pyridin, Furo (3,2-c) pyridin, Thieno (2,3-b)pyridin, Thieno(3,2-b)pyridin, Thieno (2,3-c)pyridin, Thieno (3,2-c) pyridin, Thieno (3,4-b)pyridin, Thieno (3,4-c)pyridin, 3-Methylisoxazolo (4,5-c)pyridin.

Das erfindungsgemäße Verfahren zur Herstellung von Verbindungen der Formel I ist dadurch gekennzeichnet, daß man

a) eine Verbindung der allgemeinen Formel II

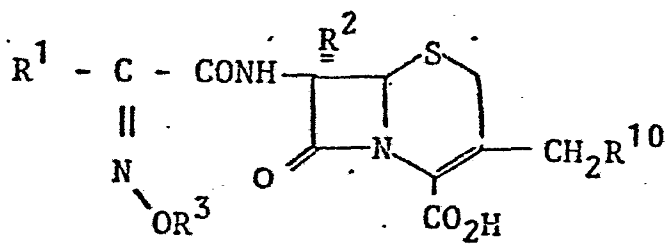

(II)

oder deren Salze, worin $R^1$, $R^2$ und $R^3$ die in Formel I genannte Bedeutung haben, und $R^{10}$ eine durch diejenige Base, die den Resten A der Formel I entspricht, austauschbare Gruppe bedeutet, mit dieser Base umsetzt und

α) eine gegebenenfalls vorhandene Schutzgruppe abspaltet und

β) falls erforderlich, das erhaltene Produkt in ein physiologisch verträgliches Säureadditionssalz überführt,

oder

b) eine Verbindung der allgemeinen Formel III,

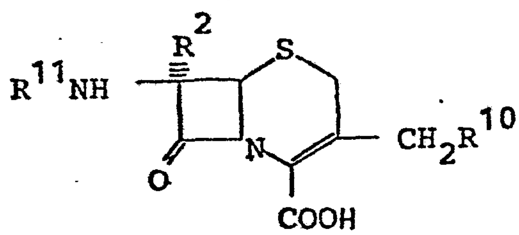

(III)

worin $R^2$ und $R^{10}$ die vorstehend für Formel II genannte Bedeutung haben und $R^{11}$ für Wasserstoff oder eine Aminoschutzgruppe steht, mit der Base, die dem in

Formel I definierten Rest A zugrunde liegt, umsetzt unter Bildung der Verbindung der allgemeinen Formel IV,

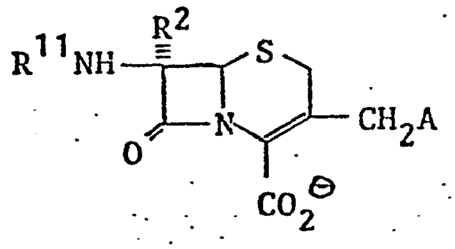

(IV),

in der $R^2$, $R^{11}$ und A die oben genannte Bedeutung haben und

α) eine gegebenenfalls vorhandene Aminoschutzgruppe abspaltet und

β) die Verbindung IV, worin $R^{11}$ Wasserstoff bedeutet, entweder als solche oder in Form eines reaktionsfähigen Derivates mit einer 2-syn-Oxyiminoessigsäure der allgemeinen Formel V,

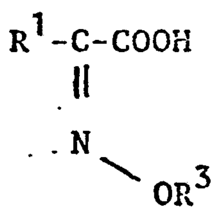

(V)

worin $R^1$ und $R^3$ die genannte Bedeutung besitzen, oder mit einem an der Carbonylgruppe aktivierten Derivat dieser Verbindung umsetzt und

a) eine gegebenfalls vorhandene Schutzgruppe abgespaltet und

ß) falls erforderlich, das erhaltene Produkt der allgemeinen Formel I in ein physiologisch verträgliches Säure-

0137442

additionssalz überführt.

Soll die Herstellung der Verbindungen der allgemeinen Formel I durch nucleophilen Austausch von $R^{10}$ in den Verbindungen der allgemeinen Formel II mit der Base, die dem in Formel I definierten Rest A zugrundeliegt, erfolgen, so kommen als Reste $R^{10}$ insbesondere in Betracht Acyloxyreste von niederen aliphatischen Carbonsäuren vorzugsweise mit 1 bis 4 C-Atomen, wie z.B. Acetoxy oder Propionyloxy, insbesondere Acetoxy die gegebenenfalls substituiert sein können, wie z.B. Chloracetoxy oder Acetylacetoxy. Für $R^{10}$ kommen auch andere Gruppen in Betracht, wie beispielsweise Halogen, insbesondere Chlor, Brom oder Jod, oder Carbmoyloxy.

Erfindungsgemäß werden bei der nucleophilen Austauschreaktion Ausgangsverbindungen der allgemeinen Formel II, in denen $R^{10}$ für Acetoxy steht, eingesetzt, oder deren Salze z.B. ein Natrium- oder Kaliumsalz. Die Reaktion wird in einem Lösungsmittel, vorzugsweise in Wasser oder in einem Gemisch aus Wasser und einem mit Wasser leicht mischbaren organischen Lösungsmittel, wie z.B. Aceton, Dioxan, Acetonitril, Dimethylformamid, Dimethylsulfoxid oder Äthanol durchgeführt. Die Reaktionstemperatur liegt im allgemeinen im Bereich von etwa 10 bis etwa 100°C, vorzugsweise zwischen 20 und 80°C. Die Basenkomponente wird in Mengen zugegeben, die zwischen etwa äquimolaren Mengen und einem bis zu etwa 15-fachen Überschuß liegt. Der Austausch des Restes $R^{10}$ wird durch Anwesenheit von Neutralsalzionen im Reaktionsmedium erleichtert. Insbesondere werden etwa 10 bis etwa 80 Äquivalente Kaliumjodid, Kaliumthiocyanat oder Natriumthiocyanat zugeben. Die Reaktion wird vorteilhaft in der Nähe des Neutralpunktes, vorzugsweise bei einem pH-Wert im Bereich von etwa 5 bis 8 durchgeführt. Liegen in den Ausgangsverbindungen der allgemeinen Formel II geschützte Aminofunktionen

vor, so eignen sich als Aminoschutzgruppen z.B. gegebenenfalls substituierte Alkyl, wie beispielsweise tert.-Butyl, tert.Amyl, Benzyl, p-Methoxy-benzyl, trityl, Benzhydryl, vorzugsweise Trityl; Trialkylsilyl, wie beispielsweise Trimethylsilyl; gegebenenfalls substituiertes aliphatisches Acyl, wie z.B. Formyl, Chloracetyl, Bromacetyl, Trichloracetyl und Trifluoracetyl, vorzugsweise Formyl; oder gegebenenfalls substituiertes Alkoxycarbonyl, wie beispielsweise Trichloräthoxycarbonyl, Benzyloxycarbonyl oder tert.-Butyloxycarbonyl, vorzugsweise tert.-Butyloxycarbonyl und Benzyloxycarbonyl; oderDimethylaminomethylen. Die Schutzgruppen sind literaturbekannt und können im Prinzip auch für den Schutz von OH und COOH-Funktionen verwendet werden. Die Schutzgruppe kann nach derAustauschreaktion in an sich bekannter Weise abgespalten werden, z.B. die Tritylgruppe mittels einer Carbonsäure, wie. z.B. Essigsäure, Trifluoressigsäure, Ameisensäure oder die Benzyl oxycarbonylgruppe hydrogenolytisch.

Die Reaktionsprodukte der Formel I können aus der Reaktionsmischung in üblicher Weise, z.B. durch Gefriertrocknen der Wasserphase, Chromatographie oder auch durch Ausfällen als schwerlösliches Salz, beispielsweise als Hydrojodid- oder Hydrothiocyanatsalz, isoliert werden.

Die nucleophile Austauschreaktion an Verbindungen der allgemeinen Formel II, kann auch so erfolgen, daß die Reaktion in Gegenwart der den Resten A entsprechenden Base und von Tri-$C_1$-$C_4$-alkyljodsilanen, wie z.B. Trimethyl- oder Triäthyljodsilan,vorgenommen wird. Dabei kann so verfahren werden, daß die Verbindung II zuerst mit Trimethyljodsilan nach den im folgenden genannten Reaktionsbedingungen zur Reaktion gebracht wird, die gebildete Verbindung II mit $R^{10}$ = J isoliert und anschließend mit der Base umgesetzt wird, oder die 3-$CH_2$ J-Verbindung in situ durch Zugabe der Base zur Reaktion gebracht wird. Eine bevorzugteAusführungsform ist in den deutschen Patentanmeldungen P 33 16 796.6, P 33 16 797.4 und P 33 16

798.2 beschrieben. Sie besteht darin, daß zu einer Lösung oder Suspension der Verbindung II in einem geeigneten Lösungsmittel die dem Rest A entsprechende Base zugegeben wird, gefolgt von Trimethyljodsilan . Statt Trimethyljodsilan kann beispielsweise auch eine Reaktionsmischung aus Jod und Hexamethyldisilan, die vorher bei Temperaturen zwischen etwa 60 und 120°C in literaturbekannter Weise zur Reaktion gebracht wurden, wobei Trimethyljodsilan entsteht, verwendet werden. Statt Trimethyljodsilan kann mit demselben guten Ergebnis auch Triethyljodsilan, das in literaturbekannter Weise hergestellt wird, verwendet werden.
Die Reaktion wird ausgeführt bei Temperaturen zwischen etwa -5° und +100°c, vorzugsweise zwischen +10° und +80°C.

Geeignete inerte aprotonische Lösungsmittel sind z.B. chlorierte Kohlenwasserstoffe, wie Methylenchlorid, Chloroform, Dichlorethan, Trichlorethan, Tetrachlorkohlenstoff, oder Niederalkylnitrile, wie Acetonitril oder Propionitril oder Frigene; insbesondere ist Methylenchlorid ein hervorragendes Lösungsmittel.

Die dem Rest A entsprechende Base wird in mindestens stöchiometrischer Menge bis zu einem zwanzigfachen Überschuß zugegeben, vorzugsweise wird mit solchen Mengen gearbeitet, daß die freiwerdende Jodwasserstoffmenge gebunden wird und noch mindestens 1 Mol, vorzugsweise 2-5 Mol der Base für die Substitution zur Verfügung stehen.

Da neben der auszutauschenden Gruppe $R^{10}$ in der Ausgangsverbindung II auch andere funktionelle Gruppe, wie z.B. die Carboxylgruppe, mit Trimethyljodsilan reagieren, wird letzteres in mindestens doppeltem bis zu fünfzehnfachen, vorzugsweise in drei- bis zehnfachem Überschuß zugegeben.

Derartige funktionelle Gruppen können auch durch Zugabe eines Silylierungsmittels wie z.B. Bistrimethylsilyl-

acetamid, N-Methyl-N-trimethylsilyltrifluoracetamid, Bistrimethylsilyltrifluoracetamid, Trimethylchlorsilan, Hexamethyldisilazan, Bistrimethylsilyharnstoff, vorsilyliert werden, entweder ohne oder in Gegenwart einer Base, vorzugsweise der gewünschten, der Gruppe A zugrundeliegenden Base in den vorstehend beschriebenen Mengen. Anschließend wird Trimethyljodsilan in mindestens stöchiometrischer Menge oder auch im Überschuß, vorzugsweise in einem doppelten bis zu einem zehnfachen Überschuß zugegeben.

Die Reaktionsprodukte der Formel I können beispielsweise nach Zugabe von Wasser oder wäßrigen Mineralsäuren, z.B. verdünnter HCL, HBr, HJ oder $H_2SO_4$, aus der wäßrigen Phase in üblicher Weise, z.B. durch Gefriertrocknen der Wasserphase, Chromatographie, oder Fällung durch Zugabe von organischen Lösungsmitteln, isoliert werden. Vorzugsweise werden die Reaktionsprodukte durch Ausfällen aus der wäßrigen Lösung in Form eines schwerlöslichen Salzes, beispielsweise eines Hydrojodidsalzes, isoliert.

Für den Fall, daß $R^{10}$ für eine Carbamoyloxygruppe steht, wird die Austauschreaktion analog durchgeführt. Steht $R^{10}$ für Brom, so erfolgt der Austausch in literaturbekannter Weise.
Nach der Verfahrensvariante b) werden die Verbindungen der allgemeinen Formel IV aus den Verbindungen der allgemeinen Formel III, z.B. der 7-Aminocephalosporansäure oder der 3-Jodmethyl-7-amino-ceph-3-em-4-carbonsäure, oder deren geschützte reaktonsfähige Derivate in analoger Weise wie vorstehend für die Verbindungen der Formel II beschreiben, darstellt.

Die Verbindung der allgemeinen Formel IV wird anschließend mit Carbonsäuren der allgemeinen Formel V acyliert, wobei eine gegebenenfallls zu besseren Durchführung der Austauschreaktion vorhandene Aminoschutzgruppe $R^{11}$, bei-

spielsweise eine tert.-Butyl, Benzyl, Trityl, Benzhydryl, Formyl, Trichloracetyl, Trifluoracetyl, Sulfo oder Dimethylaminomethylen-Gruppe, in an sich bekannter Weise vorher abgespalten wird.

Werden die Carbonsäuren der allgemeinen Formel V selbst als Acylierungsmittel eingesetzt, so wird zweckmäßig in Gegenwart eines Kondensationsmittels, beispielsweise eines Carbodiimids, wie beispielsweise N,N'-Dicylohexylcarbodiimid, gearbeitet.

Die Aktivierung der Carbonsäuren der allgemeinen Formel V kann in besonders günstiger Weise durch Behandlung mit bestimmten Carbonsäurenamiden und beispielsweise Phosgen, Phosphorpentachlorid, Tosylchlorid, Thionylchlorid oder Oxalylchlorid erfolgen, wie sie in der deutschen Patentschrift 28 04040 beschrieben wird.

Als aktivierte Derivate der Carbonsäuren der allgemeinen Formel V eignen sich insbesondere auch Halogenide, vorzugsweise Chloride, die in an sich bekannter Weise durch Behandlung mit Halogenierungsmitteln, wie z.B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel V eignen sich ferner die Anhydride und gemischten Anhydride, Azide und aktivierten Ester und Thioester, vorzugsweise mit p-Nitrophenol, 2,4-Dinitrophenol, Methylencyanhydrin, N-Hydroxysuccinimid und N-Hydroxyphthalimid, insbesondere diejenigen mit 1-Hydroxybenzotriazol, 6-Chlor-1-hydroxybenzotriazol, und 2-Mercaptobenzthiazol. Als gemischte Anhydride sind besonders geeignet solche mit niederen Alkansäuren, wie z.B. Essigsäure, und besonders bevorzugt solche mit substituierten Essigsäuren, wie z.B. Trichloressigsäure, Pivalinsäure oder Cyanessigsäure. Besonders geeignet sind aber auch die ge-mischten Anhydride mit Kohlensäurehalbestern, die man beispielsweise durch Umsetzung der Carbonsäuren der Formel V, in den die Aminogruppe geschützt ist, mit Chlorameisensäure-benzylester,-p-nitrobenzylester,-iso-butylester, -ethylester oder -allylester gewinnt. Die akivierten Derivate können als isolierte Substanzen, aber auch in situ umgesetzt werden.
Im allgemeinen erolgt die Umsetzung der Cephemderderivate der allgemeinen Formel IV mit einer Carbonsäure der allgemeinen Formel V oder einem aktivierten Derivat derselben in Gegenwart eines inerten Lösungsmittels. Insbesondere eignen sich chlorierte Kohlenwasserstoffe, wie vorzugsweise Methylenchlorid und Chloroform, Ether, wie z.B. Diethylether, Tetrahydrofuran und Dioxan; Ketone, wie vorzugsweise Aceton und Butanon; Amide, wie vorzugsweise Dimethylformamid und Dimethylacetamid oder Pyridin. Es kann sich auch als vorteilhaft erweisen, Gemische der genannten Lösungsmittel zu verwenden. Dies ist oftmals dann der Fall, wenn die Cephemverbindung der allgemeinen Formel IV mit einem in situ erzeugten aktivierten Derivat einer Carbonsäure der Formel V umgesetzt wird.

Es ist dabei nicht unbedingt erforderlich, die Verbindungen der allgemeinen Formel IV zu isolieren. Die Reaktion kann auch so durchgeführt werden, daß man Verbindungen der allgemeinen Formel III, beispielsweise 7-Aminocephalosporansäure oder 3-Jodmethyl-7-amino-ceph-3-em-4-carbonsäure oder deren geschützte reaktionsfähige Derivate in einem geeigneten Lösungsmittel mit der dem Rest A in der allgemeinen Formel IV entsprechenden Base umsetzt und die entstandene Verbindung der allgemeinen Formel IV in situ zu den Verbindungen der allgemeinen Formel I acyliert.

Die Umsetzung von Cephemverbindungen der Formel IV mit Carbonsäuren der Formel V bzw. deren aktivierten Derivaten kann in einem Temperaturbereich von etwa -80 bis etwa +80°C vorzugsweise zwischen etwa -30 und +50°C, insbesondere

jedoch zwischen etwa -20°C und Raumtemperatur erfolgen.

Die Reaktionsdauer hängt von den Reaktanten, der Temperatur und dem Lösungsmittel bzw. dem Lösungsmittelsgemisch ab und liegt normalerweise zwischen etwa 1/4 und etwa 72 Stunden.

Die Reaktion mit Säurehalogeniden kann gegebenenfalls in Gegenwart eines säurebindenden mittels zur Bindung des freigesetzten Halogenwasserstoffs durchgeführt werden. Als solche eignen sich insbesondere tertiäre Amine, wie z.B. Triethylamin oder Dimethylanilin; anorganische Basen, wie z.B. Kaliumcarbonat oder Natriumcarbonat; Alkylenoxide, wie z.B. Propylenoxid.
Auch die Anwesenheit eines Katalysators, wie z.B. von Dimethylaminopyridin, kann gegebenenfalls von Vorteil sein.

Liegt in den Verbindungen der allgemeinen Formel IV die Aminogruppe in Form eines reaktionsfähigen Derivates vor, so kann es sich um ein solches handeln, wie es aus der Literatur für Amidierungen bekannt ist. So kommen beispielsweise Silylderivate in Betracht, die bei der Umsetzung von Verbindungen der allgemeinen Formel IV mit einer Silylverbindung gebildet werden, wie z.B. Trimethylchlorsilan oder Bistrimethylsilylacetamid. Wird die Umsetzung mit einer solchen, an der Aminogruppe aktivierten Verbindung durchgeführt, so ist es zweckmäßig, die Reaktion in einem inerten Lösungsmittel, wie z.B. Methylenchlorid, Tetrahydrofuran oder Dimethylformamid, durchzuführen.

Als physiologisch verträgliche Säureadditionssalze der Verbindungen der allgemeinen Formel I seien beispielsweise erwähnt solche mit Chlorwasserstoffsäure, Bromwasserstoffsäure, Salpetersäure, Phosphorsäure, Schwefelsäure oder organischen Säuren, wie z.B. Methansulfonsäure, p-Toluolsuolfonsäure oder Maleinsäure.

Die erfindungsgemäß erhaltenen Verbindungen der allgemeinen Formel I und ihre physiologisch verträglichen Säureadditionssalze zeigen bemerkenswert gute antibakterielle Wirksamkeit sowohl gegen grampositive als auch gramnegative bakterielle Keime.

Auch gegen penicillinase - und cephlalosporinasebildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindunggemäßen Verbindungen charakterisiert sind.

Die erfindungsgemäßen Produkte können auch in Kombination mit anderen Wirkstoffen, beispielsweise aus der Reihe der Penicilline, Cephalosporine oder Aminoglykoside zur Anwendung kommen.

Die Verbindungen der allgemeinen Formel I und ihre physiologisch verträglichen Säureadditionssalze können oral, intramuskulär oder intravenös verabfolgt werden. Arzneipräparate, die eine oder mehrere Verbindungen der allgemeinen Formel I als Wirkstoff enthalten, können hergestellt werden, indem man die Verbindungen der Formel I mit einem oder mehreren pharmakologisch verträglichen Trägerstoffen oder Verdünnungsmittel, wie z.B. Füllstoffen, Emulgatoren, Gleitstoffen, Geschmackskorrigentien, Farbstoffen oder Puffersubstanzen vermischt und in eine geeignete galenische Zubereitungsform bringt, wie beispielsweise Tabletten, Dragees, Kapseln, oder einer zu parentalen Applikation geeigneten Suspension oder Lösung.

Als Träger- oder Verdünngungsmittel seien beispielsweise erwähnt Traganth, Milchzucker, Talkum, Agar-Agar, Polyglykole, Äthanol und Wasser. Puffersubstanzen sind beispielsweise organische Verbindugnen wie z.B. N,N'-Dibenzyl äthylendiamin, Diäthanolamin, Äthylendiamin, N-Methylglucamin, N-Benzylphenäthylamin, Diäthylamin, Tris(hydroxymethyl)aminomethan, oder anorganische Verbindungen, wie z.B. Phospatpuffer, Natriumcarbonat, Natiumbicarbonat. Für die parenterale Applikation kommen vorzugsweise Suspensionen oder Lösungen in Wasser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der allgemeinen Formel I oder ihrer physiologisch verträglichen Säureadditionssalze liegen bei etwa 0,4 bis 20 g/Tag, vorzugsweise bei 0,5 bis 4 g/Tag, für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 100 bis 500 mg, enthalten kann.

Die folgenden Ausführungsbeispiele für erfindungsgemäß herstellbare syn-Verbindungen dienen zur weiteren Erläuterung der Erfindung, schränken sie jedoch nicht darauf ein.

## Beispiel 1

**3-[(2.3-Cyclopentenopyridinio)-methyl]-7-[2-(1H-pyrazol-3-yl)-2-syn-methoxyimino-acetamido]-ceph-3-em-4-carboxylat**

### Verfahren $a_1$

1.27 g 7-[2-(1H-pyrazol-3-yl)-2-syn-methoxyimino-acetamido]-cephalosporansäure werden in 30 ml Wasser und 10 ml Aceton mit 2.9 g Kaliumrhodanid bei pH ca. 7 durch Zugabe von 3 ml 1N NaOH gelöst, mit 1.08 g 2.3-Cyclopentenopyridin versetzt und 5 h bei 60° C Kolbeninnentemperatur gerührt. Nach Abkühlen auf Raumtemperatur wird zweimal mit wenig Essigester extrahiert, die wässrige Phase gefriergetrocknet. Der Rückstand wird in ca. 10 ml eines Gemisches aus Wasser/Aceton (1:3) gelöst und mit Aceton/Wasser (3:1) über Kieselgel chromatographiert. Die Produktfraktionen werden lyophilisiert. Man erhält 0.12 g der Titelverbindung als farblosen amorphen Feststoff.

$^1$H-NMR ($CF_3CO_2D$) : $\delta$=2.35-2.90 (m, 2H, Cyclopenten-H), 3.20-3.80 (m, 6H, Cyclopenten-H und $SCH_2$), 4.41 (s, 3H, $OCH_3$), 5.40 und 5.95 (AB, J=18Hz, 2H, $CH_2N^{\oplus}$), 5.43 (d, J=4Hz, 1H, ß-Lactam-H), 6.13 (d, J=4Hz, 1H, ß-Lactam-H), 7.23 und 8,07 (2d, J=2Hz, 2 Pyrazol-H), 7.60-8.60 ppm (m, 3H, Pyridin-H).

Die folgenden in Tabelle 1 aufgeführten Beispiele, die der allgemeinen Formel I mit $R^2$ = Wasserstoff entsprechen, werden analog zu Beispiel 1 hergestellt und nach Chromatographie als amorphe Feststoffe erhalten:

| Beispiel | $R^1$ | $R^3$ | A | $^1H$-NMR in $CF_3CO_2D$: δ(ppm) = |
|---|---|---|---|---|
| 2 | 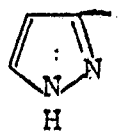 | $CH_3$ | 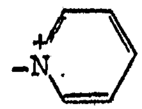 | 3.43 und 3.96 (dd, 2H, J=18Hz, $CH_2S$), 4.41 (s, 3H, $OCH_3$), 5.49 und 6.23 (AB, J=18Hz, $CH_2-N^{\oplus}$), 5.40 und 6.14 (je d, J=4Hz, 2 ß-Lactam-H), 7.23 und 8.18 (2d, J=2Hz, 2 Pyrazol-H), 8.05 - 9.15 (m, 5H, Pyridin-H). |
| 3 | 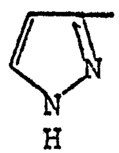 | $CHF_2$ | 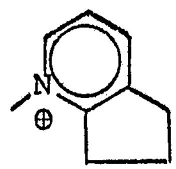 | 2.30 - 2.80 (m, 2H, Cyclopenten-H), 3.15 - 3.85 (m, 6H, $SCH_2$ und Cyclopenten-H), 5.20 - 6.20 (m, 4H, $CH_2-N^{\oplus}$ und 2 Lactam-H), 6.67 (t, J=72Hz, 1H, $CHF_2$), 7.23 und 8.18 (jew. d, J=2Hz, 2H, Pyrazol-H), 7.65 - 8.60 (m, 3H, Pyridin-H). |

Tabelle 1 (Forts.)

| Beispiel | $R^1$ | $R^3$ | A | $^1H$-NMR in $CF_3CO_2D$; δ (ppm) = |
|---|---|---|---|---|
| 4 | 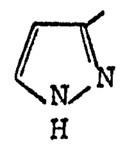 | $CH_2COONa$ | 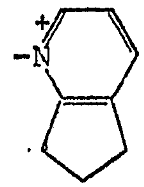 | 2.35 - 2.80 (m, 2H, Cyclopenten-H), 3.20 - 3.75 (m, 6H, Cyclopenten-H, $SCH_2$), 5.63 (s, 2H, $CH_2COONa$), 5.40 (d, J=4Hz, 1H, ß-Lactam), 5.43 und 5.97 (AB, J=18Hz, 2H, $CH_2N^{\oplus}$), 6.19 (d, J=4Hz, 1H, ß-Lactam), 7.25 und 8.20 (2d, J=2Hz, 2 Pyrazol-H), 7.65-8.60 (m, 3H, Pyridin-H). |
| 5 | 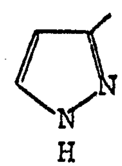 | $CH_2COONa$ | 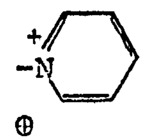 | 3.39 und 3.90 (AB, J=18Hz, 2H, $CH_2S$), 5.20 (s, 2H, $CH_2COONa$), 5.41 und 6.16 (jew. d, J=4Hz, 2 ß-Lactam-H), 5.48 und 6.20 (AB, J=18Hz, 2H, $CH_2N^{\oplus}$), 7.25 und 8.20 (2 d, J=2Hz, 2 Pyrazol-H), 8,05 - 9.15 (m, 5H, Pyridin-H). |

Tabelle 1 (Forts.)

| Beispiel | $R^1$ | $R^3$ | A | $^1H$-NMR in $CF_3CO_2D$: δ (ppm) = |
|---|---|---|---|---|
| 6 | 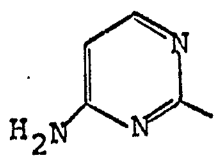 | $-CH_2-CF_3$ | 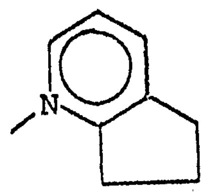 | 2.25 - 2.9 (m, 2H, Cyclopenten-H), 3.1 - 3.9 (m, 6H, $SCH_2$ und Cyclopenten-H), 4.7 (q, J=9Hz, 2H, $CH_2-CF_3$), 5.2 - 6.3 (m, 4H, $CH_2-N^{\oplus}$ und 2 Lactam-H), 6.9 und 8.15 (jew. d., J=7Hz, 2 Pyrimidin-H), 7.6 - 8.7 (m, Pyridin-H). |

0137442

## Beispiel 7

## 7-[2-(4-Aminopyrimidin-2-yl)-2-syn-ethoxyimino-acetamido]-3-(2.3-cyclohexenopyridiniomethyl)-ceph-3-em-4-carboxylat

### Verfahren $a_2$

0.3 g (0.56 mmol) 2-(4-Aminopyrimidin-2-yl)-2-syn-ethoxyimino-acetamido-cephalosporansäure Trifluoracetat werden in 10 ml abs. Methylenchlorid suspendiert, 0.66 g (5 mmol) 2.3-Cyclohexenopyridin und danach 0.57 ml (4 mmol) Trimethyljodsilan zugegeben, unter Schutzgas 2 Stunden zum Rückfluß erhitzt, nach Abkühlen 20 ml Wasser zugesetzt, mit Natriumbicarbonat neutralisiert, noch zweimal mit Methylenchlorid extrahiert, eingeengt und mit Aceton/Wasser (3:1) an Kieselgel ("Lobar-B"-Säule, Fa. Merck) chromatographiert. Die Produktfraktionen werden gefriergetrocknet und man erhält 0.045 g eines farblosen amorphen Feststoffes.

$^1$H-NMR ($CF_3CO_2D$) : δ = 1.45 (t, J=7Hz, 3H, OEt), 1.8-2.4 (m, 4H, Cyclohexen-H), 2.9-4.0 (m, 6H, $SCH_2$ und Cyclohexen-H), 4.63 (q, J=7Hz, 2H, OEt), 5.16-6.26 (m, 4H, $CH_2N^{\oplus}$ und 2 Lactam-H), 6.89 (d, J=6Hz, 1H, Pyrimidin-H), 7.5-8.7 ppm (m, 4H, Pyridin-H und 1 Pyrimidin-H).

Beispiel 8

7-[2-(4-Aminopyrimidin-2-yl)-2-syn-methoxyimino-acetamido]-3- (2.3-cyclohexenopyridiniomethyl) -ceph-3-em-4-carboxylat

0.34 g (0.6 mmol) 2-(4-Aminopyrimidin-2-yl)-2-syn-methoxy-imino-acetamido]-cephalosporansäure werden in 15 ml $CH_2Cl_2$ mit 0.86 g (6.5 mmol) 2.3-Cyclohexenopyridin und 0.74 ml (5.2 mmol) Trimethyljodsilan analog Beispiel 7 umgesetzt. Ausbeute nach Chromatographie: 0.03 g Lyphylisat.

$^1$H-NMR ($CF_3CO_2D$): δ = 1.8 - 2.33 (m, 4H, Cyclohexen-H), 2.9 - 3.3 (m, 4H, Cyclohexen-H), 3.1 - 4.0 (AB, ~ 2H, $SCH_2$, z. T. überlagert mit Cyclohexen-H), 4.33 (s, 3H, OMe), 5.2 - 6.25 (m, 4H, $CH_2N^{\oplus}$ und 2 Lactam-H), 6.88 (d, J=7Hz, 1H, Pyrimidin-H), 7.58 - 8.66 ppm (m, 4H, Pyridin-H und 1 Pyrimidin-H).

Beispiel 9

7-[2-(4-Aminopyrimidin-2-yl)-2-syn-methoxyimino-acetamido]-3-[(3-ethylpyridinio)-methyl]-ceph-3-em-4-carboxylat

Herstellung erfolgt analog Beispiel 7 im 0.6 mmol Maßstab. Ausbeute nach Chromatographie: 0.055 g.

$^1H$-NMR ($CF_3CO_2D$): δ = 1.43 (t, J=7Hz, 3H, Et), 2.7 (q, J=7Hz, 2H, Et), 3.2 - 4.0 (AB, J=18Hz, 2H, $SCH_2$), 4.33 (s, 3H, OMe), 5.2 - 6.3 (m, 4H, Lactam-H und $CH_2$-$N^{\oplus}$), 6.89 (d, J=7Hz, 1H, Pyrimidin-H), 7.8 - 8.95 ppm (m, 5H, Pyridin-H und Pyrimidin-H).

Beispiel 10

7-[2-(4-Aminopyrimidin-2-yl)-2-syn-methoxyimino-acetamido]-3-(1-chinolinio -methyl)-ceph-3-em-4-carboxylat

Durchführung analog Beispiel 7 im 2 mmol-Maßstab.
Ausbeute: 0.25 g Lyophylisat.

$^1H$-NMR ($CF_3CO_2D$): δ = 3.1 - 4.0 (AB, J=18Hz, 2H, $SCH_2$), 4.34 (s, 1H, OMe), 5.3 - 6.4 (m, 4H, 2 Lactam-H und $CH_2N^{\oplus}$), 6.9 (d, J=7Hz, 1H, Pyrimidin-H), 7.95 - 8.65 (m, 6H, Chinolin-H und 1 Pyrimidin-H), 8.95 - 9.4 ppm (m, 2H, Chinolin-H).

Beispiel 11

7-[2-(4-Aminopyrimidin-2-yl)-2-syn-ethoxyimino-acetamido]-3-[(4-cyclopropylpyridinio) methyl]-ceph-3-em-4-carboxylat

Verfahren $b_1$

0.49 g (2 mmol) 2-(4-Aminopyrimidin-2-yl)2-syn-ethoxyimino-essigsäure-Hydrochlorid werden zusammen mit 0.16 ml (2 mmol)

0137442

Pyridin in 10 ml N,N-Dimethylformamid (DMF) gelöst, nach Zugabe von 0.34 g (2.2 mmol) 1-Hydroxybenzotriazolhydrat und 0.49 g (2.4 mmol) N,N'-Dicylohexylcarbodiimid wird 3 Stdn. bei Raumtemperatur gerührt und vom ausgefallenen Harnstoff abfiltriert. Das Filtrat wird bei $0^{o}$ C zu einer Lösung aus 0.81 g (2 mmol) 7-Amino-3-[(4-cylopropylpyridinio)-methyl]-ceph-3-em-4-carbonsäure-Dihydrochlorid in 10 ml DMF/1 ml Wasser getropft, über Nacht bei $0^{o}$ C gerührt und bei Vollständigkeit der Reaktion (DC-Kontrolle) in ca. 500 ml Ether gegossen. Man dekantiert, rührt noch zweimal mit Aceton aus, nimmt mit Aceton/Wasser (3:1) unter Zusatz von etwas Bicarbonat auf und chromatographiert das Filtrat an Kieselgel (Säule "Lobar B", Fa. Merck) mit Aceton/Wasser (3:1). Die Produktfraktionen werden nach Entfernen von Aceton i. Vak. gefriergetrocknet.

Ausbeute: 0.19 g eines farblosen amorphen Feststoffes.

$^{1}$H-NMR ($CF_3CO_2D$): δ = 1.06-1.93 (m, 7H, OEt und $CH_2$ von Cyclopropyl), 2.0-2.53 (m, 1H, CH von Cyclopropyl), 3.2-4.0 (AB, J=18Hz, 2H, $SCH_2$), 4.6 (q, J=7Hz, 2H, OEt), 5.0-6.3 (m, 4H, $CH_2N^{\oplus}$ und 2 Lactam-H), 6.9 und 8.1 (jew. d, J=7Hz, 2 Pyrimidin-H), 7.6 und 8.66 ppm (AA'BB', J=6.5Hz, 4H, Pyridin-H).

0137442

Die folgenden, in Tabelle 2 aufgeführten Beispiele, die der allgemeinen Formel I mit $R^2$= Wasserstoff entsprechen, werden analog zu Beispiel 11 hergestellt und nach Chromatographie und Gefriertrocknen als amorphe Feststoffe erhalten.

Tabelle 2

| Beispiel | $R^1$ | $R^3$ | A | $^1H$-NMR in $CF_3CO_2D$: δ (ppm) = |
|---|---|---|---|---|
| 12 | 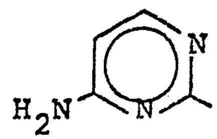 | Me | 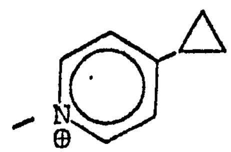 | 1.08-1.95 (m, 4H, $CH_2$ von Cyclopropyl), 2.0-2.5 (m, 1H, CH von Cyclopropyl), 3.2-3.95 (AB, 2H, $SCH_2$), 4.33 (s, 3H, OMe), 5.0-6.3 (m, 4H, $CH_2-N^{\oplus}$ und 2 Lactam-H), 6.93 und 8.13 (jew. d, J=7hz, 2 Pyrimidin-H), 7.66 und 8.66 (AA'BB', 4H, Pyridin-H). |
| 13 | 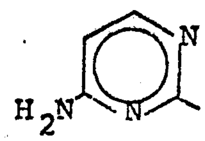 | Et | 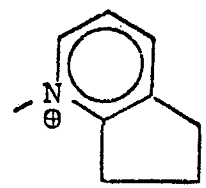 | 1.46 (t, J=7Hz, 3H, OEt), 2.25-2.9 (m, 2H, Cyclopenten-H), 3.1-3.9 (m, 6H, $SCH_2$ und Cyclopenten-H), 4.6 (q, J=7Hz, 2H, OEt), 5.2-6.3 (m, 4H, $CH_2-N^{\oplus}$ und 2 Lactam-H), 6.9 und 8.1 (jew. d, J=7Hz, 2 Pyrimidin-H), 7.6-8.67 (m, Pyridin-H). |

Tabelle 2 (Forts.)

| Beispiel | $R^1$ | $R^3$ | A | $^1H$-NMR in $CF_3CO_2D$: δ (ppm) = |
|---|---|---|---|---|
| 14 | 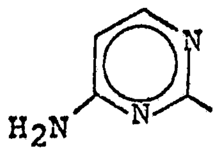 | Me | 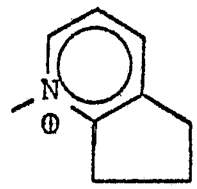 | 2.23-2.9 (m, 2H, Cyclopenten-H), 3.1-4.0 (m, 6H, $SCH_2$ und Cyclopenten-H), 4.33 (s, 3H, OMe), 5.13-6.24 (m, 4H, $CH_2N^{\oplus}$ und 2 Lactam-H), 6.9 (d, J=7Hz, 1 Pyrimidin-H), 7.6-8.7 (m, 4H, Pyridin-H und 1 Pyrimidin-H). |
| 15 | 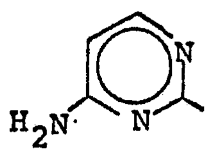 | Et | 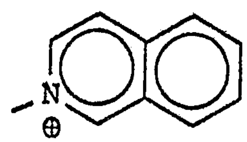 | 1.43 (t, 3H, OEt), 3.2-4.0 (AB, J=18Hz, 2H, $SCH_2$), 4.6 (q, 2H, OEt), 5.33-6.56 (m, 4H, $CH_2N^{\oplus}$ und 2 Lactam-H), 6.7 (d, J=7Hz, 1 Pyrimidin-H), 7.9-8.8 (m, 7H, Isochinolin-H und 1 Pyrimidin-H), 9.75 (sb, 1 Isochinolin-H). |

Tabelle 2 (Forts.)

| Beispiel | $R^1$ | $R^3$ | A | $^1H$-NMR in $CF_3CO_2D$: δ (ppm) = |
|---|---|---|---|---|
| 16 | 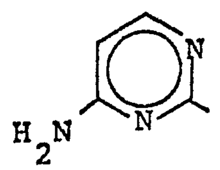 | Me | 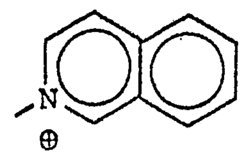 | 3.25-4.05 (AB, J=18Hz, 2H, $SCH_2$), 4.3 (s, 3H, OMe), 5.2-6.55 (m, 4H, $CH_2-N^{\oplus}$ und 2 Lactam-H), 6.9 (d, J=7Hz, 1 Pyrimidin-H), 7.8-8.85 (m, 7H, Isochinolin-H und 1 Pyrimidin-H), 9.76 (sb, 1 Isochinolin-H). |
| 17 | 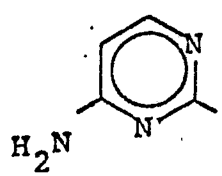 | Et | 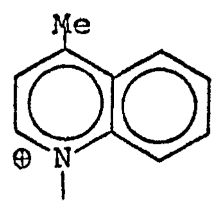 | 1.45 (t, J=7Hz, 3H, OEt), 3.13 (s, 3H, Lepidin-$CH_3$), 3.2-4.0 (AB, J=18Hz, 2H, $SCH_2$), 4.6 (q, J=7Hz, 2H, OEt), 5.22-6.4 (m, 4H, $CH_2N^{\oplus}$ und 2 Lactam-H), 6.9 (d, J=7Hz, 1 Pyrimidin-H), 7.83-8.67 (m, 6H, 5 Lepidin-H und 1 Pyrimidin-H), 9.04 (d, $J_{2,3}$=6Hz, 1H, Lepidin-2-H). |

Tabelle 2 (Forts.).

| Beispiel | $R^1$ | $R^3$ | A | $^1$H-NMR in $CF_3CO_2D$: δ (ppm) = |
|---|---|---|---|---|
| 18 | 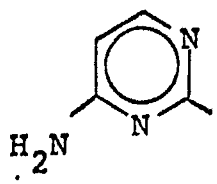 | Me | 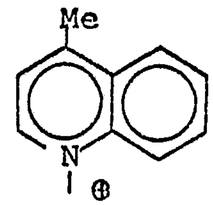 | 3.13 (s, 3H, Lepidin-$CH_3$), 3.05-3.95 (AB, J=18Hz, 2H, $SCH_2$), 4.33 (s, 3H, OMe), 5.25-6.55 (m, 4H, $CH_2$-$N^{\oplus}$ und 2 Lactam-H), 6.9 (d, J=7Hz, 1H, Pyrimidin-H), 7.83-8.67 (m, 6H, 5 Lepidin-H und 1 Pyrimidin-H), 9.03 (d, $J_{2,3}$=6Hz, 1H, Lepidin-2-H). |
| 19 | 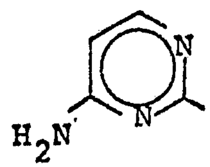 | Et | 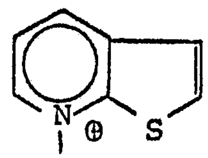 | 1.46 (t, J=7Hz, 3H, OEt), 3.2-4.05 (AB, J=18Hz, 2H, $SCH_2$), 4.6 (q, J=7Hz, 2H, OEt), 5.2-6.4 (m, 4H, $CH_2$-$N^{\oplus}$ und 2 Lactam-H), 6.9 (d, J=7Hz, 1 Pyrimidin-H), 7.66-9.2 (m, 6H, aromat. H). |

Tabelle 2 (Forts.)

| Beispiel | $R^1$ | $R^3$ | A | $^1H$-NMR in $CF_3CO_2D$: δ (ppm) = |
|---|---|---|---|---|
| 20 | 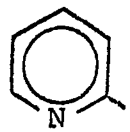 | Me | 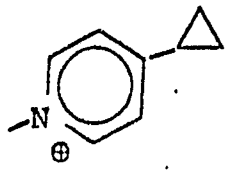 | 1.08-1.95 (m, 4H, $CH_2$ von Cyclopropyl), 2.0-2.5 (m, 1H, CH von Cyclopropyl), 3.2-4.05 (AB, J=19Hz, 2H, $SCH_2$), 4.6 (s, 3H, OMe), 5.03-6.3 (m, 4H, $CH_2N^{\oplus}$ und 2 Lactam-H), 7.65 (AA' von AA'BB', 2 Pyridin-H), 8.0-9.07 (m, 6 Pyridin-H). |
| 21 | 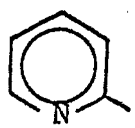 | Me | 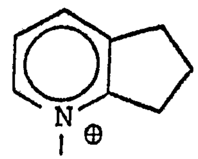 | 2.2-2.9 (m, 2H, Cyclopenten-H), 3.1-3.95 (m, 6H, $SCH_2$ und Cyclopenten-H), 4.5 (s, 3H, OMe), 5.0-6.25 (m, 4H, $CH_2N^{\oplus}$ und 2 Lactam-H), 7.66-9.1 (m, 7H, Pyridin-H). |
| 22 | 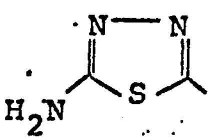 | Et | 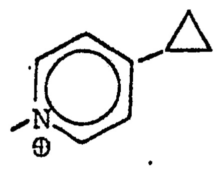 | 1.1-1.95 (m, 7H, $CH_2$ von Cyclopropyl und OEt bei 1.45), 2.0-2.5 (m 1H, CH von Cyclopropyl), 3.15-4.0 (AB, J=18Hz, 2H, $SCH_2$), 4.58 (q, J=7Hz, 2H, OEt), 5.0-6.3 (m, 4H, $CH_2$-$N^{\oplus}$ und 2 Lactam-H), 7.65 und 8.66 (AA'BB', 4 Pyridin-H). |

Tabelle 2 (Forts.)

| Beispiel | $R^1$ | $R^3$ | A | $^1H$-NMR in $CF_3CO_2D$: δ (ppm) = |
|---|---|---|---|---|
| 23 | 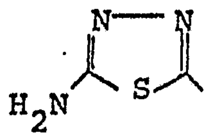 | Et | 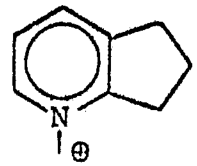 | 1.46 (t, J=7Hz, 3H, OEt), 2.2-2.9 (m, 2H, Cyclopenten-H), 3.1-3.95 (m, 6H, $SCH_2$ und Cyclopenten-H), 4.6 (q, J=7Hz, 2H, OEt), 5.15-6.23 (m, 4H, $CH_2$-$N^{\oplus}$ und 2 Lactam-H), 7.56-8.8 (m, 3 Pyridin-H). |
| 24 | 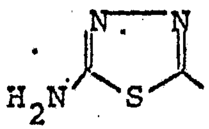 | Et | 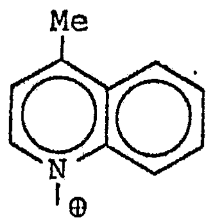 | 1.45 (t, J=7Hz, 3H, OEt), 3.0-3.95 (5H, s von Lepidin-$CH_3$ bei 3.15 überlagert mit AB und $SCH_2$), 4.45 (q, J=7Hz, 2H, $SCH_2$), 5.0-6.3 (m, 4H, $CH_2N^{\oplus}$ und 2 Lactam-H), 7.8-9.3 (m, 6 Lepidin-H). |
| 25 | 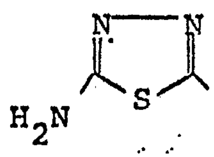 | Me | 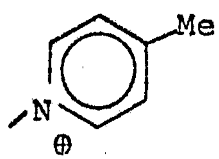 | 2.75 (s, 3H, Pyridin-Me), 3.15-3.95 (AB, J=18Hz, 2H $SCH_2$), 4.46 (s, 3H, OMe), 5.15-6.25 (m, 4H, -$CH_2$-$N^{\oplus}$ und 2 Lactam-H), 7.93 und 8.8 (AA'BB', 4Pyridin-H). |

Tabelle 2 (Forts.)

| Beispiel | $R^1$ | $R^3$ | A | $^1H$-NMR in $CF_3CO_2D$: δ (ppm) = |
|---|---|---|---|---|
| 26 | 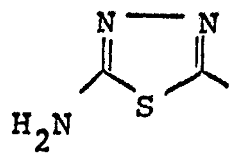 | Me | 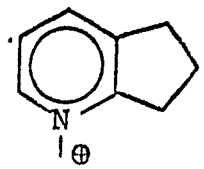 | 2.25-2.80 (m, 2H, Cyclopenten-H), 3.10-3.95 (m, 6H, Cyclopenten-H und $SCH_2$), 4.36 (s, 3H, OMe), 5.2-6.20 (m, 4H, $CH_2N^{\oplus}$ und 2 Lactam-H), 7.65-8.70 (m, 3 Pyridin-H). |
| 27 | 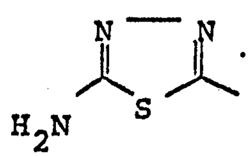 | Me | 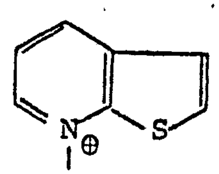 | 3.15-3.95 (AB, J=18Hz, 2H, $SCH_2$), 4.46 (s, 3H, OMe), 5.20-6.30 (m, 4H, $CH_2N^{\oplus}$ und 2 Lactam-H), 7.66-9.33 (m, 5H, Ar-H). |
| 28 | 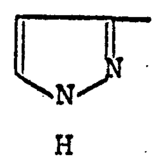 | Me | 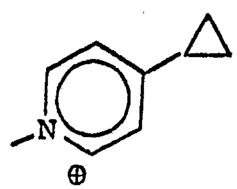 | 1.1-1.95 (m, 4H, $CH_2$ von Cyclopropyl ), 2.0-2.5 (m, 1H, CH von Cyclopropyl), 3.1-4.0 (AB, J=18Hz, 2H, $SCH_2$), 5.1-6.3 (m, 4H, $CH_2N^{\oplus}$ und 2 Lactam-H), 4.40 (s, 3H, OMe), 7.25 und 8.18 (jew. d, 1H, J=2.2Hz, Pyrazol-H), 7.65 und 8.65 (AA'BB', 4 Pyridin-H). |

Beispiel 29

7-[2-Carboxymethyloxyimino-2-(1H-pyrazol-3-yl)-acetamido]-3-(4-cylopropylpyridinio)methyl-ceph-3-em-4-carbonsäure-Trifluoracetat

Durchführung analog Beispiel 11 mit Aktivester aus 2 mmol 2-(tert.-Butoxycarbonylmethoxyimino)-2-(1H-pyrazol-3-yl)-essigsäure und 0.18 g (2 mmol) 7-Amino-3-[1-(4-cyclopropyl-pyridinio)-methyl]-ceph-3-em-4-carbonsäure-Dihydrochlorid. Spaltung des tert.-Butylesters mit Trifluoressigsäure (30 min bei Raumtemperatur) nach chromatographischer Reinigung. Ausbeute: 60 mg Trifluoracetat

$^1$H-NMR ($CF_3CO_2D$): δ = 1.1-1.95 (m, 4H, $CH_2$ von Cyclopropyl ), 2.0-2.5 (m, 1H, CH von Cyclopropyl), 3.05-4.05 (AB, J=18Hz, 2H, $SCH_2$), 5.0-6.45 (m, 6H, $CH_2$ $N^{\oplus}$, 2 Lactam-H und $CH_2COOH$ bei 5.22), 7.25 und 8.2 (jew. d, J=2.2Hz, 2 Pyrazol-H), 7.65 und 8.66 ppm (AA'BB', 4 Pyridin-H).

Beispiel 30

7-[2-(4-Aminopyrimidin-2-yl)-2-syn-ethoxyimino-acetamido]-3-[1-(3-methylpyridinio)-methyl]-ceph-3-em-4-carboxylat

Verfahren $b_2$

Lösung A:

1.02 g (3 mmol) 7-Amino-3-jodmethyl-ceph-3-em-4-carbonsäure werden in 75 ml abs. Methylenschlorid suspendiert, mit 2.22 ml (9 mmol) N, O-Bis-(trimethylsilyl)-acetamid (BSA) in Lösung gebracht, und nach Zugabe von 0.88 ml (9 mmol) 3-Picolin 6 Stunden bei Raumtemperatur gerührt.

Lösung B:

0.62 g (2.5 mmol) syn-2-Ethoxyimino-2-(4-aminopyrimidin-2-yl)-essigsäure-Hydrochlorid werden zusammen mit 0.2 ml (2.5 mmol) Pyridin in 12.5 ml N,N-Dimethylformamid (DMF) gelöst, 0.38 g (2.5 mmol) 1-Hydroxybenzotriazolhydrat und 0.57 g (2.75 mmol) N,N'-Dicyclohexylcarbodiimid zugesetzt, 3 Stunden gerührt, und vom Harnstoff abfiltriert.

Die Lösung B wird bei $0^{o}$ C zu obiger Methylenchlorid-Lösung A getropft, über Nacht bei Raumtemperatur gerührt, 15 ml Methanol zugesetzt nach 30 Minuten das Methylenchlorid abdestilliert und die DMF-Lösung in ca. 200 ml Diethylether gegossen. Der Niederschlag wird abgesaugt, mit Ether gewaschen, mit Aceton/Wasser unter Zusatz von Natriumbicarbonat gelöst und an Kieselgel in Analogie zu Beispiel 11 chromatrographiert.
Ausbeute: 0.13 farbloser amorpher Feststoff.

$^{1}$H-NMR ($CF_3$-$CO_2$D):δ = 1.46 ( t, J=7Hz, 3H, OEt), 2.68 (s, 3H, Pyridin-Me), 3.16-4.0 (AB, J=18Hz, 2H, $SCH_2$), 4.63 (q, J=7Hz, 2H, OEt), 5.1-6.4 (m, 4H, $CH_2N^{\oplus}$ und 2 Lactam-H), 6.9 (d, J=7Hz, 1 Pyrimidin-H), 7.8-9.0 ppm (m, 5H, Pyridin-H und 1 Pyrimidin-H).

Die folgenden, in Tabelle 3 aufgeführten Beispiele, die der allgemeinen Formel I mit $R^2$= Wasserstoff entsprechen, werden analog zu Beispiel 30 hergestellt und nach Chromatographie und Gefriertrocknen als amorphe Feststoffe erhalten.

Tabelle 3

| Beispiel | $R^1$ | $R^3$ | A | $^1$H-NMR in $CF_3CO_2D$: δ (ppm) = |
|---|---|---|---|---|
| 31 | 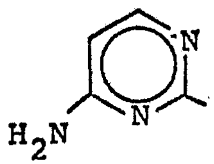 | Me | 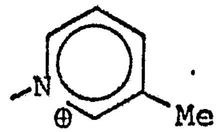 | 2.66 (s, 3H, Pyridin-Me), 3.2-4.05 (AB, J=19Hz, 2H, $SCH_2$), 4.3 (s, 3H, OMe), 5.1-6.43 (m, 4H, $CH_2N^{\oplus}$ und 2 Lactam-H), 6.89 (d, J=7Hz, 1 Pyrimidin-H), 7.83-8.95 (m, 5H, Pyridin-H und 1 Pyrimidin-H). |
| 32 | 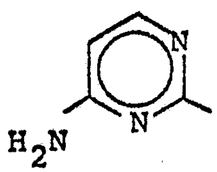 | Et | 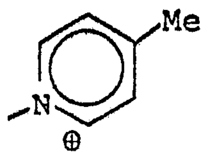 | 1.46 (t, J=7Hz, 3H, OEt), 2.76 (s, 3H, Pyridin-Me), 3.16-4.05 (AB, J=19Hz, 2H, $SCH_2$), 4.6 (q, J=7Hz, 2H, OEt), 5.1-6.4 (m, 4H $CH_2N^{\oplus}$ und 2 Lactam-H), 6.89-8.93 (je ein d, J=7Hz, 2 Pyrimidin-H), 7.93 und 8.33 (AA'BB', 4 Pyridin-H). |
| 33 | 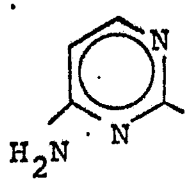 | Me | 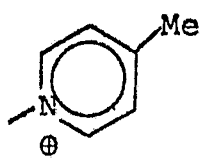 | 2.76 (s, 3H, Pyridin-Me), 3.2-4.0 (AB, J=18Hz, 2H, $SCH_2$), 4.33 (s, 3H, OMe), 5.06-6.33 (m, 4H, $CH_2N^{\oplus}$ und 2 Lactam-H), 6.9 und 8.13 (jew. d, J=7Hz, 2 Pyrimidin-H), 7.93 und 8.81 (AA'BB', 4 Pyridin-H). |

<u>Tabelle 3</u> (Forts.)

| Beispiel | $R^1$ | $R^3$ | A | $^1H$-NMR in $CF_3CO_2D$: δ (ppm) = |
|---|---|---|---|---|
| 34 | 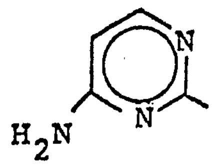 | Et | 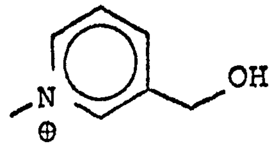 | 1.46 (t, J=7Hz, 3H, OEt), 3.2-4.1 (AB, J=18Hz, 2H, $SCH_2$), 4.59 (q, J=7Hz, 2H, OEt), 5.0-6.5 (m, 6H, $CH_2N^{\oplus}$, 2 Lactam-H und $CH_2$-OH bei 5.2), 6.9 (d, J=7Hz, 1 Pyrimidin-H), 7.93-9.3 (m, 5H, Pyridin-H und 1 Pyrimidin-H). |
| 35 | 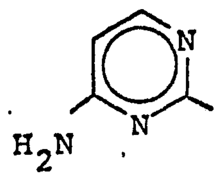 | Et | 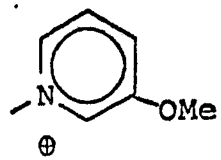 | 1.46 (t, J=7Hz, 3H, OEt), 3.15-4.0 (AB, J=19Hz, 2H, $SCH_2$), 4.05 (s, 3H, Pyridin-OMe), 4.6 (q, J=7Hz, 2H, OEt), 5.05-6.4 (m, 4H, $CH_2N^{\oplus}$ und 2 Lactam-H), 6.9 (d, J=7Hz, 1 Pyrimidin-H), 7.86-8.8 (m, 5H, Pyridin-H und 1 Pyrimidin-H). |
| 36 | 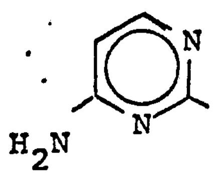 | Et | 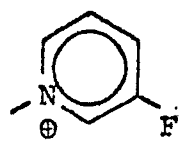 | 1.45 (t, J=7Hz, 3H, OEt), 3.25-4.05 (AB, J=19Hz, 2H, $SCH_2$), 4.6 (q, J=7Hz, 2H, OEt), 5.2-6.47 (m, 4H, $CH_2N^{\oplus}$ und 2 Lactam-H), 6.9 (d, J=7Hz, 1 Pyrimidin-H), 6.97-9.25 (m, 5H, Pyridin-H und 1 Pyrimidin-H). |

Tabelle 3 (Forts.)

| Beispiel | $R^1$ | $R^3$ | A | $^1H$-NMR in $CF_3CO_2D$: δ (ppm) ≙ |
|---|---|---|---|---|
| 37 | 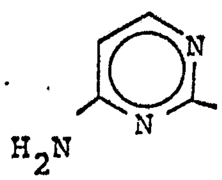 | Et | 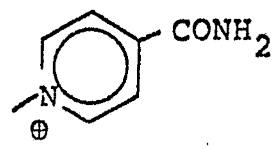 | 1.45 (t, J=7Hz, 3H, OEt), 3.2-4.0 (AB, 2H, $SCH_2$), 4.6 (q, J=7Hz, 2H, OEt), 5.2-6.5 (m, 4H, $CH_2N^{\oplus}$ und 2 Lactam-H), 6.9 und 8.1 (jew. d, J=7Hz, 2 Pyrimidin-H), 8.6 und 9.23 (AA'BB', 4 Pyridin-H). |
| 38 | 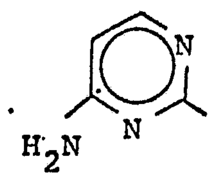 | Me | 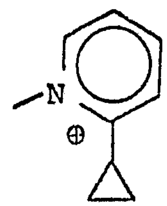 | 1.1-1.86 (m, 4H, $CH_2$ von Cyclopropyl), 2.1-2.6 (m, 1H, CH von Cyclopropyl), 3.2-4.0 (AB, 2H, $SCH_2$), 4.33 (s, 3H, OMe), 5.05-6.35 (m, 4H, $CH_2N^{\oplus}$ und 2 Lactam-H), 6.9 (d, J=7Hz, 1 Pyrimidin-H), 7.65-8.86 (m, 5H, Pyridin-H und 1 Pyrimidin-H). |
| 39 | 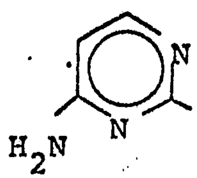 | Et | 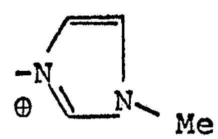 | 1.47 (t, J=7Hz, 3H, OEt), 3.25-4.0 (AB, J=18Hz, 2H, $SCH_2$), 4.03 (s, 3H, NMe), 4.63 (q, J=7Hz, 2H, OEt), 5.0-6.25 (m, 4H, $CH_2N^{\oplus}$ und 2 Lactam-H), 6.9 und 8.13 (jew. d, J=7Hz, 2 Pyrimidin-H), 7.27-7.7 (m, 2 Imidazol-H), 8.47 (m, 1 Imidazol-H). |

Tabelle 3 (Forts.)

| Beispiel | $R^1$ | $R^3$ | A | $^1H$-NMR in $CF_3CO_2D$: δ (ppm) = |
|---|---|---|---|---|
| 40 | 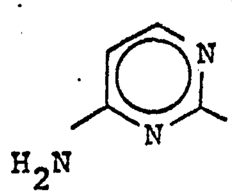 | Et | 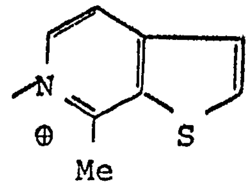 | 1.46 (t, J=7Hz, 3H, OEt), 3.05-4.0 (4H, AB von $SCH_2$ überlagert mit Pyridin-Me als s bei 3.26), 4.63 (q, J=7Hz, 2H, OEt), 5.2-6.25 (m, 4H, $CH_2N^{\oplus}$ und 2 Lactam-H), 6.9 (d, J=7Hz, 1 Pyrimidin-H), 7.6-8.7 (m, 5H, aromat. H). |
| 41 | 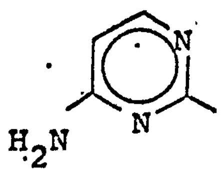 | Et | 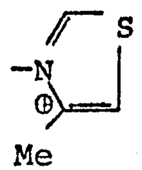 | 1.47 (t, J=7Hz, 3H, OEt), 2.75 (s, 3H, Thiazol-Me), 3.2-4.05 (AB, J=18Hz, 2H, $SCH_2$), 4.62 (q, J=7Hz, 2H, OEt), 5.15-6.25 (m, 4H, $CH_2N^{\oplus}$ und 2 Lactam-H), 6.9 und 8.15 (jew. d, J=7Hz, 2 Pyrimidin-H), 7.82 und 9.78 (jew. d, J=2Hz, 2 Thiazol-H). |
| 42 | 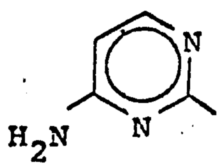 | Et | 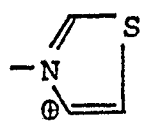 | 1.45 (t, J=7Hz, 3H, OEt), 3.15-4.1 (AB, J=18Hz, 2H, $SCH_2$), 4.6 (q, J=7Hz, 2H, OEt), 5.13-6.22 (m, 4H, $CH_2$-$N^{\oplus}$ und 2 Lactam-H), 6.9 und 8.15 (jew. d, J=7Hz, Pyrimidin-H, z. T. überlagert mit Thiazol), 8.2, 8.5 und 10.04 (jew. m, 1H, Thiazol-H). |

Beispiel 43

7-[2-(4-Aminopyrimidin-2-yl)-2-syn-ethoxyimino-acetamido]-3-(trimethylammoniomethyl)-ceph-3-em-4-carboxylat

0.68g (2 mmol) 7-Amino-3-jodmethyl-ceph-3-em-4-carbonsäure werden in 40 ml DMF suspendiert, eine Lösung aus 0.35 g (6 mmol) Trimethylamin in 3 ml DMF zugesetzt und 4 Stunden bei Raumtemperatur gerührt. Acylierung mit HOBT-Ester erfolgt wie in Beispiel 30 beschrieben mit 2 mmol Aktivester-Lösung in DMF. Nach Beendigung der Reaktion wird in Ether gegossen und der Rückstand wie beschrieben chromatographiert. Ausbeute: 95 mg amorpher Feststoff.

$^1$H-NMR ($CF_3CO_2D$): δ = 1.47 (t, J=7Hz, 3H, OEt), 3.05-4.0 (~11H, $SCH_2$ überlagert mit s von $NMe_3$ bei δ = 3.45), 4.3-4.9 (m, 4H, OEt überlagert mit $CH_2N^{\oplus}$), 5.46 und 5.95 (jew. d, J=4Hz, 2 Lactam-H), 6.9 und 8.1 ppm (jew. d, J=7Hz, 2 Pyrimidin-H).

Die folgenden, in Tabelle 4 aufgeführten Beispiele, die der allgemeinen Formel I mit $R^2$= Wasserstoff entsprechen, werden analog zu Beispiel 43 hergestellt und nach Chromatographie und Gefriertrocknen als amorphe Feststoffe erhalten.

Tabelle 4

| Beispiel | $R^1$ | $R^3$ | A | $^1H$-NMR in $CF_3CO_2D$: δ (ppm) = |
|---|---|---|---|---|
| 44 | 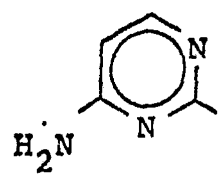 | Et | $-\overset{\oplus}{N}Et_3$ | 1.26-1.75 (m, 12H, $CH_3$ von $NEt_3$ und OEt), 3.1-4.0 (m, ~8H, $SCH_2$ und $CH_2$ von $NEt_3$), 4.37-4.85 (m, 4H, OEt überlagert mit $CH_2N^{\oplus}$), 5.48 und 5.97 (jew. d, J=4.2Hz, 2 Lactam-H), 6.9 und 8.12 (jew.d, J=7Hz, 2 Pyrimidin-H). |
| 45 | 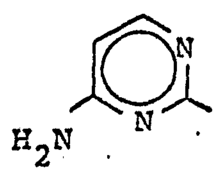 | Et | 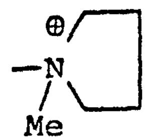 | 1.48 (t, J=7Hz, 3H, OEt), 2.1-2.65 (m, 4H, Pyrrolidin-$CH_2$), 3.0-4.05 (m, 9H, $SCH_2$, Pyrrolidin-N-$CH_2$ und N-Me bei δ=3.22), 4.35-4.85 (m, 4H, $\overline{CH_2N}^{\oplus}$ und OEt), 5.48 und 5.98 (jew. d, J=4.2Hz, 2 Lactam-H), 6.9 und 8.12 (jew. d, J=7Hz, 2 Pyrimidin-H). |
| 46 | 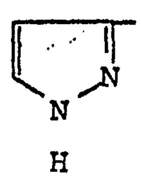 | Me | $-\overset{\oplus}{N}Me_3$ | 3.36 (9H, s, $NMe_3$), 3.80 (br. s, 2H, $SCH_2$), 4.43 (3H, s, $OCH_3$), 4.53 und 5.03 (2H, AB, J=14Hz, $\overline{CH_2-N^{\oplus}}$), 5.46 und 5.96 (jew. d, J=5Hz, 2 Lactam-H), 7.23 und 8.1 (jew. d, J=2Hz, 2 Pyrazol-H). |

Tabelle 4 (Forts.)

| Beispiel | $R^1$ | $R^3$ | A | $^1H$-NMR in $CF_3CO_2D$: δ (ppm) = |
|---|---|---|---|---|
| 47 | 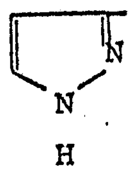 | Me | $-\overset{\oplus}{N}Et_3$ | 1.50 (br. t, 9H, $CH_2CH_3$), 3.53 (br. q, 2H, $CH_2CH_3$), 3.80 (br. s, 2H, $SCH_2$), 4.43 (s, 3H, $OCH_3$), 4.46-5.0 (AB, J=14Hz, 2H, $CH_2-N^{\oplus}$), 5.43 und 5.96 (jew. d, J=5Hz, 2 Lactam-H), 7.23 und 8.13 (jew. d, J=2Hz, 2 Pyrazol-H). |

Beispiel 48:

7-[2-(2-Aminooxazol-4-yl)-2-syn-methoxyimino-acetamido]-3-(isochinoliniomethyl)-ceph-3-em-4-carboxylat

1. Stufe: (Verfahren $b_2$)

3-(Isochinoliniomethyl)-7-[2-syn-methoxyimino-2-(2-tert-butyloxycarbonylaminooxazol-4-yl)-acetamido]-ceph-3-em-4-carboxylat:

Lösung A:

680 mg (2 mmol) 7-Amino-3-jodmethyl-ceph-3-em-4-carbonsäure werden mit 1.48 ml (6 mmol) N,O-Bis-(trimethylsilyl)-acetamid in 50 ml Methylenchlorid in Lösung gebracht. Nach Zugabe von 0.776 g (6 mmol) Isochinolin wird 4 1/2 Stunden bei Raumtemperatur gerührt.

Lösung B:

570 mg (2 mmol) 2-syn-Methoxyimino-2-[2-tert-butyloxycarbonylamino-oxazol-4-yl]-essigsäure werden in 8 ml DMF gelöst und nach Zugabe von 288 mg 1-Hydroxybenzotriazolhydrat und 412 mg N,N'-Dicyclohexylcarbodiimid 3 1/2 Stunden bei Raumtemperatur gerührt und vom ausgefallenen N,N'-Dicyclohexylharnstoff abfiltriert.

Unter Eiskühlung wird Lösung B zu Lösung A getropft, 1 1/2 Stunden bei 0° C gerührt und über Nacht bei 4° C belassen. Die Reaktionslösung wird im Vakuum eingeengt, in 8 ml Aceton/$H_2O$ (3:1) gelöst, mit Natriumbicarbonatlösung auf pH 6.0 eingestellt und über Silicagel mit Aceton/$H_2O$ (3:1) chromatographiert. Die Produktfraktionen werden vereinigt und gefriergetrocknet. Es werden 200 mg eines hellen Pulvers gewonnen.

2. Stufe:

150 mg des Produktes aus Stufe 1 werden bei 0° C mit 3 ml Trifluoressigsäure gerührt. Nach 3 Stunden wird 20 ml Diethylether zugegeben und im Vakuum eingeengt. Der erhaltene Sirup wird in 5 ml Aceton/$H_2O$ (2:1) gelöst, mit Natriumbicarbonat auf pH 6.0 gestellt und über Silicagel mit Aceton/$H_2O$ (2:1) chromatographiert. Die das Produkt enthaltenden Fraktionen werden vereinigt und ergeben nach Gefriertrocknen 80 mg der Titelverbindung als amorphen Feststoff.

$^1$H-NMR (DMSO-$d_6$): δ = 3.3 (br. s, 2H, $SCH_2$), 3.8 (s, 3H, OMe), 4.9-5.7 (4H, d von 6-H (J=5Hz) bei 5.0, dd von 7-H (J=5 und 9Hz) bei 5.6 sowie AB von $CH_2$-$N^{\oplus}$ bei 5.5), 6.8 (br. s, 2H, $NH_2$), 7.3 (s, 1H, Oxazol-H), 8.0-8.7 und 10.2 (6H, Isochinolin-H), 9.4 ppm (d, J=9Hz, 1H, CONH).

Die folgenden, in Tabelle 5 aufgeführten Verbindungen, die der allgemeinen Formel I mit $R^2$= Wasserstoff entsprechen, werden analog Beispiel 48 hergestellt und , als amorphe Feststoffe erhalten:

Tabelle 5

| Beispiel | $R^1$ | $R^3$ | A | $^1$H-NMR (DMSO-$d_6$): δ (ppm) = |
|---|---|---|---|---|
| 49 | 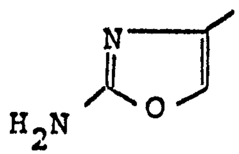 | $CH_3$ | 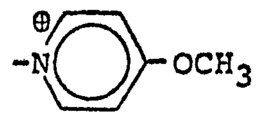 | 3.3 (br. s, $SCH_2$, überlagert mit HDO), 3.8 (s, 3H, OMe), 4.07 (s, 3H, Pyridin-OMe), 5.0-5.55 (4H, d von 6-H (J=5Hz) bei 5.03, dd von 7-H bei 5.5 und AB von $CH_2$-$N^{\oplus}$ bei 5.3), 6.8 (br. s, 2H, $NH_2$), 7.4 (s, 1 Oxazol-H), 7.6 und 9.27 (AA'BB', 4 Pyridin-H), 9.5 (d, J=8Hz, 1H, CONH). |
| 50 | 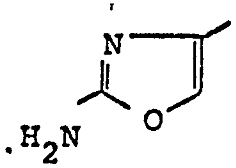 | $CH_3$ | 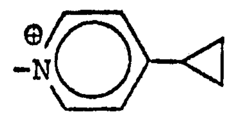 | 1.0-1.6 (m, 4H, $CH_2$ von Cyclopropyl), 1.95-2.4 (m, 1H, CH von Cyclopropyl), 3.3 (br. s, $SCH_2$, überlagert mit HDO), 3.8 (s, 3H, OMe), 4.9-5.7 (4H, ß-Lactam-H bei 5.0 und 5.5, überlagert mit AB von $CH_2$-$N^{\oplus}$), 6.8 (br. s, 2H, $NH_2$), 7.4 (s, 1 Oxazol-H), 7.78 und 9.17 (AA'BB', 4 Pyridin-H), 9.5 (d, J=9Hz, 1H, CONH). |

Tabelle 5 (Forts.).

| Beispiel | $R^1$ | $R^3$ | A | $^1$H-NMR (DMSO-$d_6$): δ (ppm) = |
|---|---|---|---|---|
| 51 | 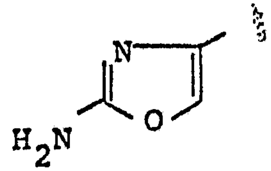 | $CH_3$ | 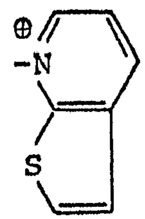 | 3.3 (br. s, 2H, $SCH_2$), 3.9 (s, 3H, OMe), 5.06 (d, J=5Hz, 1 ß-Lactam-H), 5.45-6.1 (3H, $CH_2N^{\oplus}$ überlagert mit ß-Lactam-H), 6.8 (br, s, 2H, $NH_2$), 7.4 (s, 1 Oxazol-H), 7.7-9.6 (m, 6H, Aromaten-H und CONH). |
| 52 | 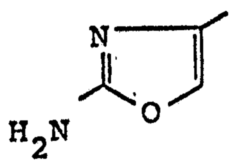 | $CH_3$ | $-\overset{\oplus}{N}(CH_3)_3$ | 3.0 (br. s, 9H, $NMe_3$), 3.3 (br. s, 2H, $SCH_2$), 3.55-4.25 ( 6H, OMe bei 3.83 überlagert mit AB von $CH_2$-$N^{\oplus}$), 5.1 (d, J=5Hz, 1 ß-Lactam-H), 5.6 (dd, J=5 und 9Hz, 1 ß-Lactam-H), 6.8 (br. s, 2H, $NH_2$), 7.47 (s, 1 Oxazol-H), 9.5 (d, J=9Hz, 1H, CONH). |

**Beispiel 53**

**7-[2(2-Aminooxazol-4-yl)-2-syn-methoxyimino-acetamido]-3-(2.3-cyclopentenopyridinio-methyl)-ceph-3-em-4-carboxylat**

215 mg (0.5 mmol) 2-syn-methoxyimino-(2-N-tritylaminooxazol-4-yl)-essigsäure, gelöst in 5 ml THF wurden mit 72 mg (0.5 mmol) 1-Hydroxybenzotriazol Monohydrat und 103 mg (0.5 mmol) N,N'-Dicyclohexylcarbodiimid versetzt. Nach 2-stündigem Rühren bei Raumtemperatur wurde filtriert und das Filtrat zu einer Lösung aus 211 mg (0.5 mmol) 7-Amino-3-(2.3-cyclopentenopyridiniomethyl)-3-ceph-em-4-carboxylat in 21 ml DMF und 0.2 ml $H_2O$ gegeben. Die Mischung wurde 36 Stunden bei + 4° C belassen, filtriert und im Vakuum eingeengt.
Zu dem so erhaltenen öligen Rückstand wurde bei 0° C 3 ml gekühlte 90 % Trifluoressigsäure gegeben. Es wurde 1 Stunde bei 0° C gerührt, im Vakuum eingeengt, mit Diethylether versetzt (10 ml) und erneut eingeengt. Der Rückstand wurde in 20 ml Aceton/$H_2O$ (3:1) aufgenommen und mit ges. $NaHCO_3$-Lösung auf pH 6.0 gestellt. Es wurde zum Trocknen eingeengt und erneut in 5 ml Aceton/$H_2O$ (3:1) aufgenommen, filtriert und an Kieselgel mit Aceton/$H_2O$ (3:1) chromatographiert. Die das Produkt enthaltenden Fraktionen wurden vereinigt und gefriergetrocknet. Es wurden 30 mg der Titelverbindung erhalten.

$^1$H-NMR ($CF_3CO_2D$): δ = 2.1-2.9 (m, 2H, Cyclopenten-H), 3.1-3.9 (m, 6 H, Cyclopenten-H und $SCH_2$), 4.3 (s, 3H, OMe), 5.2-6.6 (m, 4H, $CH_2$-$N^{\oplus}$ und 2 Lactam-H), 8.03 (s, 1 Oxazol-H), 7.7-8.7 ppm (m, 3 Pyridin-H).

Beispiel 54

7-[2-(2-Amino-4-methyl-(1.3.5)-triazin-6-yl)-2-syn- ethoxy-imino-acetamido]-3-(2.3-cyclopentenopyridiniomethyl)-ceph-3-em-4-carboxylat

a) 2-(2-Amino-4-methyl-(1.3.5)-triazin-6-yl)-2-syn-ethoxy-imino-essigsäureethylester.

10.8 g 2-Amidino-2-syn-ethoxyimino-essigsäureethylester Hydrochlorid (Herstellung s. EP 0046964) werden in 50 ml Methanol gelöst, bei $0^{o}$ C 2.54 g Natriummethanolat und nach 10 min 5.8 g Ethyl-N-Cyanacetimidat zugegeben, dann 50 Stunden bei Raumtemperatur gerührt. Nach Einengen wird mit Essigester/Wasser aufgenommen und die organische Phase nochmals mit Wasser gewaschen, dann getrocknet und eingeengt. Das Rohprodukt wird durch Chromatographie an Kieselgel mittels Essigester/Petroleter (2:1) gereinigt, wobei ein farbloses kristallines Produkt (Schmp. 141°C) resultiert.

$^{1}$H-NMR ($CDCl_3$): δ = 1.31 (t, J=7Hz, 3H, OEt), 2.43 (s, 3H, Triazin-Me), 3.88 (s, 3H, $CO_2Me$), 4.37 (q, J=7Hz, 2H, OEt), 5.85 ppm (bs, 2H, $NH_2$).

b) 2-(2-Amino-4-methyl-(1.3.5)-triazin-6-yl)-2-syn-ethoxyiminoessigsäure-Hydrochlorid

0.4 g Produkt von Beispiel 54 a) werden in 5 ml Ethanol gelöst, eine Lösung aus 0.13 g NaOH in 5 ml Wasser zugegeben, 5 Stunden bei Raumtemperatur gerührt und Ethanol i. Vak. entfernt. Man versetzt unter Eiskühlung mit 2 N HCl bis pH 1, lyophylisiert die wässrige Lösung, und extrahiert das Produkt mit Ethanol.

Nach Einengen wird das ölige Produkt bis zur vollständigen Kristallisation mit Ether ausgerührt. Zers. > 150° C

$^1$H-NMR (DMSO-$d_6$): δ = 1.25 (t, J=7Hz, 3H, OEt), 2.3 (s, 3H, Triazin-Me), 4.27 (q, J=7Hz, 2H, OEt), 5.5-6.6 ppm (bs, 4H, $NH_3^{\oplus}$ und $CO_2H$).

c) Acylierung (Verfahren $b_1$)

0.23 g (0.9 mmol) Hydrochlorid von Beispiel 54 c) werden zusammen mit 0.072 ml (0.9 mmol) Pyridin, 0.14 g (0.9 mmol) 1-Hydroxybenzotriazolhydrat und 0.2 g (1 mmol) N,N'Dicyclohexylcarbodiimid in 5 ml abs. DMF gelöst, nach 3 Stunden vom ausgefallenen Harnstoff abfiltriert und das Filtrat bei 0° C zu einer Lösung aus 0.38 g (0.9 mmol) 7-Amino-3-[1-(2.3-cyclopentenopyridinio)-methyl]-ceph-3-em-4-carbonsäure Dihydrochlorid in 5 ml DMF/2.5 ml Wasser getropft. Man rührt über Nacht bei Raumtemperatur und arbeitet wie bei Beispiel 11 beschrieben auf. Ausbeute: 50 mg Lyophylisat.

$^1$H-NMR ($CF_3CO_2D$): δ = 1.46 (t, J=7Hz, 3H, OEt), 2.23-2.9 (5H, m von Cyclopenten-H überlagert mit s von Triazin-Me bei 2.9 ppm), 3.23-3.9 (m, 6H, $SCH_2$ und Cyclopenten-H), 4.6 (q, J=7Hz, 2H, OEt), 5.09-6.3 (m, 4H, $CH_2N^{\oplus}$ und 2 Lactam-H), 7.53-8.8 ppm (m, 3H, Pyridin-H).

Beispiel 55

7-[2-(2-Amino-4-methyl-(1.3.5)-triazin-6-yl)-2-syn-methoxyimino-acetamido]-3-[(2.3-cyclopentenopyridimio)methyl]-ceph-3-em-4-carboxylat

a) 2-(2-Amino-4-methyl-(1.3.5.)-triazin-6-yl)-2-syn-methoxyimino-essigsäuremethylester:

Analog Beispiel 54 a aus 2-Amidino-2-syn-methoxyimino-essigsäuremethylester-Hydrochlorid und Ethyl-N-Cyanoacetimidat erhalten. Farblose Kristalle von Schmp. 148-149° C.

$^1$H-NMR ($CDCl_3$): δ = 2.48 (s, 3H, $CH_3$), 3.93 (s, 3H, $CO_2Me$), 4.14 (s, 3H, $OCH_3$), 5.85 ppm (bs, 2H, $NH_2$).

b) 2-(Amino-4-methyl-(1.3.5.)-triazin-6-yl)-2-syn-methoxy-iminoessigsäure-Hydrochlorid:

Aus dem Methylester (Beispiel 54 a) durch alkalische Verseifung analog Beispiel 54 b erhalten. Farblose Kristalle, Zers. > 160° C.

$^1$H-NMR (DMSO-$d_6$): δ = 2.28 (s, 3H, Triazin-Me), 3.90 (s, 3H, OMe), 3.9-5.0 (bs, COOH), 7.56 ppm (bs, 2H, $NH_2$).

c) Acylierung

Aus 124 mg (o.5 mmol) Produkt aus Beispiel 55 b werden in Analogie zur Beispiel 54 c eine Lösung des HOBT-Aktivesters in DMF hergestellt. Umsetzung mit 230 mg (0.5 mmol) 7-Amino-3-(2.3-cyclopentenopyridinio)-methyl-ceph-3-em-4-carbonsäuremonohydrojodid in DMF/Wasser (2.8/1.4 ml) während 20 Stunden bei Raumtemperatur, einengen und Chromatographie über Kieselgel ("Lobar B"-Säule) mit Aceton/Wasser (3:1) liefert 105 mg der Titelverbindung als amorphes Lyophylisat.

$^1H$-NMR ($CF_3CO_2D$): δ = 2.15-2.85 (m, 5H, 2 Cyclopenten-H, überlagert von s bei 2.78, Triazin-Me), 3.05-4.15 (m, 6H, 4 Cyclopenten-H und $SCH_2$), 4.33 (s, 3H, OMe), 5.00-6.25 (m, 4H, $CH_2N^{\oplus}$ und 2 Lactam-H), 7.60-8.65 ppm (m, 3H, Pyridin-H).

Beispiel 56:

7-[2-(5-Aminoisothiazol-3-yl)-2-syn-methoxyimino-acetamido]-3-(2,3-cyclopentenopyridiniomethyl)-ceph-3-em-4-carboxylat

Analog Beispiel 11 aus 2-(5-Aminoisothiazol-3-yl)-2-syn-methoxyiminoessigsäure und 7-Amino-3-(2,3-cyclopentenopyridiniomethyl)-ceph-3-em-4-carbonsäure in 55 % Ausbeute als amorphen Feststoff erhalten:

$^1H$-NMR ($CF_3CO_2D$): δ = 2.42-2.71 (m, 2H, Cyclopenten-H); 3.32-4.28 (m, 6H, 4 Cyclopenten-H und $SCH_2$); 4.24 (s, 3H, $OCH_3$); 5.15-6.32 (m, 4H, $CH_2N^{\oplus}$ und 2 Lactam-H); 6.95 (s, 1H, Isothiazol); 7.65-8.68 (m, 3H, Pyridin).

Beispiel 57:

7-[2-(5-Aminoisoxazol-3-yl)-2-syn-methoxyimino-acetamido]-3-(2,3-cyclopentenopyridiniomethyl)-ceph-3-em-4-carboxylat

Analog Beispiel 11 aus 2-(5-Aminoisoxazol-3-yl)-2-syn-methoxyiminoessigsäure und 7-Amino-3-(2,3-cyclopentenopyridiniomethyl)-ceph-3-em-4-carbonsäure (62 % Ausbeute) als amorphen Feststoff erhalten:

$^1H$-NMR ($CF_3CO_2D$): δ = 2.35-2.65 (m, 2H, Cyclopenten-H); 3.30-4.18 (m, 6H, 4 Cyclopenten-H und $SCH_2$), 4.22 (s, 3H, $OCH_3$); 5.05-6.40 (m, 4H, $CH_2N^{\oplus}$ und 2 Lactam-H); 7.45-8.70 (m, 4H, 1 Isoxazol-H und 3 Pyridin-H).

Die folgenden in Tabelle 6 aufgeführten Beispiele, die der allgemeinen Formel I mit $R^2$ = Wasserstoff entsprechen, werden analog Beispiel 30 bzw. 48 (Verfahren $b_2$) oder Beispiel 53 (Verfahren $b^1$) hergestellt und als amorphe Feststoffe erhalten.

Tabelle 6

| Beispiel | $R^1$ | $R^3$ | A | Verfahren | $^1$H-NMR $\delta$ (ppm) in DMSO (DMSO-$D_6$): $\delta$ (ppm) = |
|---|---|---|---|---|---|
| 58 | 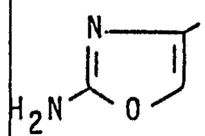 | $CH_3$ | 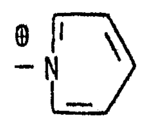 | $b^2$ | 3.3(s,2H,$SCH_2$), 3.8(s,3H,$NOCH_3$), 5.05(d,1H,C-6-H), 5.5 (m,3H,C-7-H und $CH_2N$), 6.8 (s,2H,$NH_2$), 7.43 (s,1H,Oxazol-H), 8-9.5 (m,6H, Pyridin und CONH) |
| 59 | 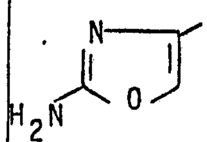 | $C_2H_5$ | 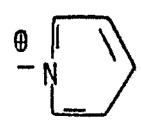 | $b^2$ | 1.17(t,3H,$CH_2\underline{CH_3}$), 3.3(s,2H,$SCH_2$), 4.07(q,2H,$\underline{CH_2}CH_3$), 5.05(d,1H,C-6-H), 5.6(m,3H,C-7H und $CH_2N$), 6.8(s,2H, $NH_2$), 7.43(s,1H,Oxazol-H), 8.0-9.7 (m,6H,Pyridin und CONH) |

| Beispiel | $R^1$ | $R^3$ | A | Verfahren | $^1H$-NMR (DMSO-$D_6$): $\delta$ (ppm) = $\delta$ (ppm) in DMSO |
|---|---|---|---|---|---|
| 60 | 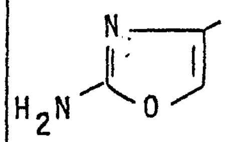 | $C_2H_5$ | 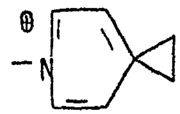 | $b^1$ | ca. 1.2 (m,8H,$CH_3$ und Cyclopropyl), 3.3(s,2H,$SCH_2$), 4.06 (q,2H,$\underline{CH_2}CH_3$), 5.0(d,1H,C-6H), 5.5 (m,3H,C-7-H und $CH_2N$), 6.77 (s,2H,$NH_2$), 7.43 (s,1H, Oxazol-H), 7.7-9.3 (m,4H,Pyridin), 9.45 (d,1H,CONH) |
| 61 | 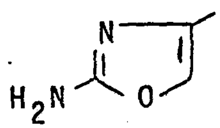 | $C_2H_5$ | 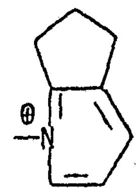 | $b^1$ | 1.3 (t,3H,$CH_2\underline{CH_3}$), 2-3.5 (m,6H,Cyclo-penteno), 3.3 (s,2H,$SCH_2$), 4.07 (q,2H, $\underline{CH_2}CH_3$), 5.05 (d,1H,C-6-H), 5.38 (br.s, 2H, $CH_2N$), 5.67 (dd,1H,C-7-H), 6.8 (s,2H,$NH_2$), 7.47 (s,1H,Oxazol), 7.5-9.3 (m,3H,Pyridin), 9.5 (d,1H, CONH) |

| Beispiel | $R^1$ | $R^3$ | A | Verfahren | $^1H$-NMR δ (ppm) in DMSO (DMSO-$D_6$): δ (ppm)= |
|---|---|---|---|---|---|
| 62 | 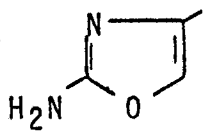 | $C_2H_5$ | 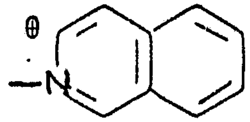 | $b^2$ | 1.15 (t,3H,$CH_2CH_3$), 3.3 (s,2H,$SCH_2$), 4.03 (q,2H,$\underline{CH_2}CH_3$), 5.05 (d,1H, C-6-H), 5.33 (AB,2H,$CH_2N$), 5.63 (dd,1H,C-7-H), 6.8 (s,2H,$NH_2$), 7.43 (s,1H,Oxazol), 8-10.3(m,12H,Isochinolin) |
| 63 | 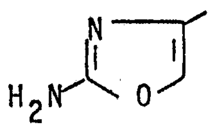 | $C_2H_5$ | 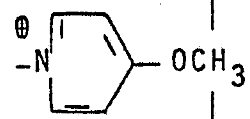 | $b^2$ | 1.3 (t,3H,$CH_2\underline{CH_3}$), 3.3 (s,2H,$SCH_2$), 4.05 (q+s,5H,$\underline{CH_2}CH_3$ und $OCH_3$), 5.05 (d,1H,C-6-H), 5.5 (m,3H,C-7-H und $CH_2N$), 6.8 (s,2H,$NH_2$), 7.45 (s,1H,Oxazol), 7.5-9.3 (m,4H, Pyridin), 9.45 (d,1H,CONH) |
| 64 | 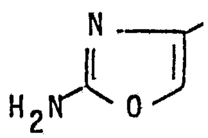 | $C_2H_5$ | $-\overset{\oplus}{N}(CH_3)_3$ | $b^2$ (Variante Beisp.43) | 1.23 (t+s,12H,$CH_2\underline{CH_3}$ und $NCH_3$), 3.3 (s,2H,$SCH_2$), 3.67 (AB,2H,$CH_2N$), 4.1 (q,2H,$\underline{CH_2}CH_3$), 5.1 (d,1H,C-6-H),5.6 (dd,1H,C-7-H), 6.8 (s,2H,$NH_2$), 7.5 (s,1H,Oxazol), 9.5 (d,1H,CONH), |

0137442

| Beispiel | $R^1$ | $R^2$ | A | Verfahren | $^1$H-NMR in ($CF_3CO_2D$): δ (ppm)= |
|---|---|---|---|---|---|
| 65 | 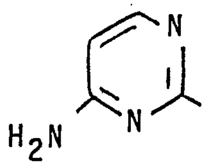 | $C_2H_5$ | 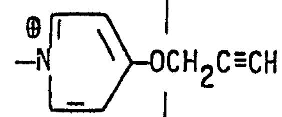 | $b^2$ | 1.47 (t,J=7Hz,3H,OEt), 2.65 (t,3x1-6Hz, 1H, Propargyl-CH), 3.2-4.15 (AB,J=18Hz,2H,$SCH_2$), 4.6 (q,J=7Hz,2H,OEt), 4.95-6.2 (m,6H, Propargyl-$CH_2$ bei 5.07, $CH_2\overset{\oplus}{N}$ und 2 Lactam-H), 6.9 und 8.1 (jew.d, J=7Hz, 2Pyrimidin-H), 7.55 und 8.78 (AA'BB', 4Pyridin-H) |
| 66 | 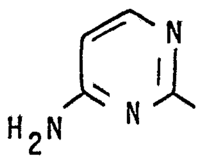 | $C_2H_5$ | 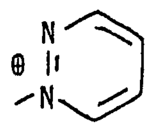 | $b^2$ | 1.45 (t,J=7Hz,3H,OEt), 4.03 (br.s, 2H,$SCH_2$), 4.6 (q,J=7Hz,2H,OEt), 5.25-6.45 (m,4H,$CH_2\overset{\oplus}{N}$ und 2 Lactam-H), 6.9 und 8.13 (jew. d, J=7Hz, 2Pyrimidin-H), 8.55(2H), 9.5 und 9.97 (jew 1H), Pyridazin-H |

| Beispiel | $R^1$ | $R^2$ | A | Verfahren | $^1H$-NMR in ($CF_3CO_2D$): δ (ppm) = |
|---|---|---|---|---|---|
| 67 | 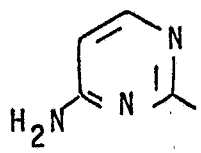 | $C_2H_5$ | 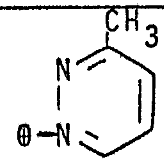 | $b^2$ | 1.47 (t, J=7Hz, 3H, OEt), 2.93 (s, 3H, Pyridazin-Me), 4.0 (br.s, 2H, $SCH_2$), 4.6 (q, J=7Hz, 2H, OEt), 5.2-6.45 (m, 4H, $CH_2\overset{\oplus}{N}$ und 2 Lactam-H), 6.9 und 8.1 (jew. d, J=7Hz, 2H, Pyrimidin-H), 8.43 (2H) und 9.75 (1H): Pyridazin-H |
| 68 | 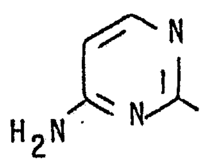 | $CH_3$ | 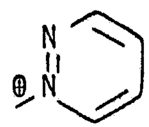 | $b^2$ | 4.0 (br.s, 2H, $SCH_2$), 4.34 (s, 3H, OMe), 5.1-6.5 (m, 4H, $CH_2\overset{\oplus}{N}$ und 2Lactam-H), 6.9 und 8.1 (jew.d, J=7Hz, 2Pyrimidin-H), 8.55 (2H), 9.5 und 9.98 (jew. 1H): Pyridazin-H |

Patentansprüche:

1. Cephemderivate der allgemeinen Formel I

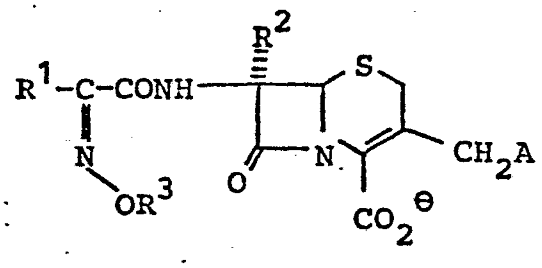

(I)

und deren physiologisch verträgliche Säureadditionssalze, worin bedeuten

$R^1$ einen 5- oder 6-gliedrigen heterocyclischen Ring, der durch folgende Formeln wiedergegeben wird

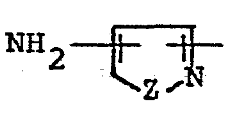 , 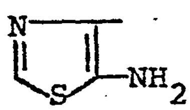 , 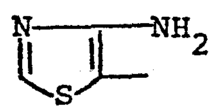 , 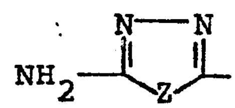 ,

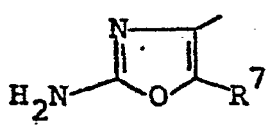 , 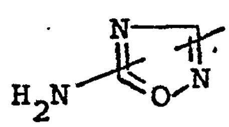 , 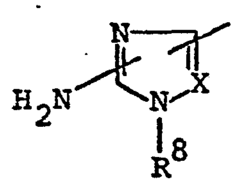 , 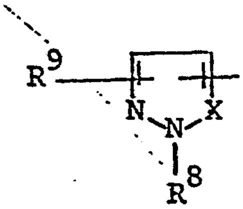 ,

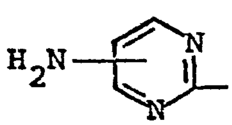 , 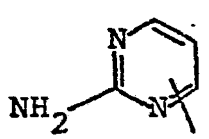 , 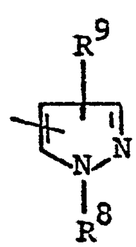 , 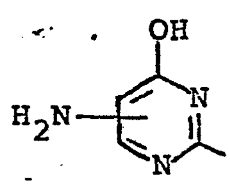 ,

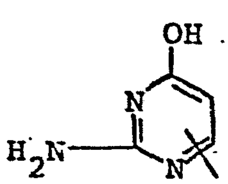 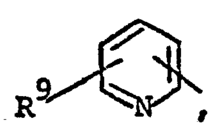 , 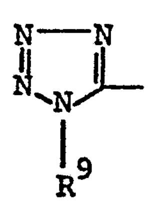 ,

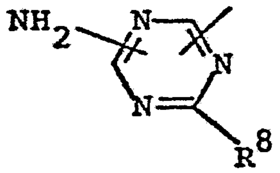 , 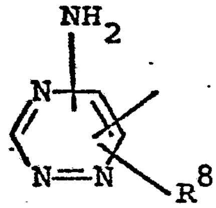

worin $R^7$ für Wasserstoff oder Halogen, $R^8$ für Wasserstoff oder einen gegebenenfalls substituierten $C_1$-$C_4$-Alkylrest, $R^9$ für Wasserstoff oder Amino, X für N oder CH, Z für O, S oder $NR^8$ steht,

$R^2$ Wasserstoff oder Methoxy

$R^3$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $-(CH_2)_n-\overset{R^4}{\underset{R^5}{(C)}}_m-R^6$ , worin m und n jeweils für 0 oder 1 steht und

$R^4$ und $R^5$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$-Alkyl- und die $C_3$-$C_7$-Cycloalkylidengruppe noch weiter ein- oder mehrfach substituiert sein können,

$R^6$ eine COOH-; CN- oder $CONH_2$-Gruppe und

A eine gegebenenfalls substituierte Ammoniogruppe, die von einem tertiären aliphatischen oder cyclischen Amin abgeleitet ist oder ein ungesättigtes, gegebenenfalls substituiertes 5-6-gliedriges heterocyclisches Kation der Formel 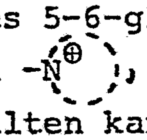, das weitere N, O oder S-Atome im Ring enthalten kann und an das mindestens ein zusätzlicher Ring ankondensiert sein kann und in der die $R^3$ O-Gruppe in syn-Position steht.

2. Verfahren zur Herstellung von Cephemverbindungen der Formel I und ihrer physiologisch verträglichen Säureadditionssalze, dadurch gekennzeichnet, daß man

a) eine Verbindung der allgemeinen Formel II

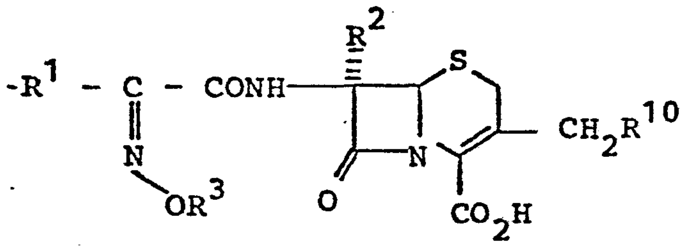

(II)

oder deren Salze, worin $R^1$, $R^2$ und $R^3$ die in Formel I genannte Bedeutung haben, und $R^{10}$ eine durch diejenige Base, die den Resten A der Formel I entspricht, austauschbare Gruppe bedeutet, mit dieser Base umsetzt und

α) eine gegebenenfalls vorhandene Schutzgruppe abspaltet und

β) falls erwünscht, das erhaltene Produkt in ein physiologisch verträgliches Säureadditionssalz überführt,

oder

b) eine Verbindung der allgemeinen Formel III,

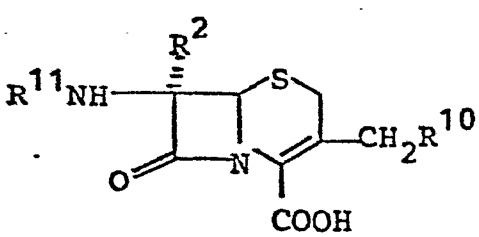

(III)

worin $R^2$ und $R^{10}$ die vorstehend für Formel II genannte Bedeutung haben und $R^{11}$ für Wasserstoff oder eine Aminoschutzgruppe steht, mit der Base, die dem in Formel I definierten Rest A zugrunde liegt, umsetzt unter Bildung der Verbindung der allgemeinen Formel IV,

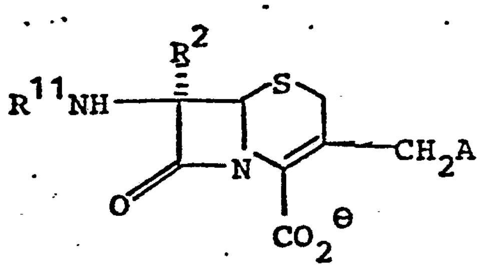

(IV),

in der $R^2$, $R^{11}$ und A die oben genannte Bedeutung haben und

α) eine gegebenenfalls vorhandene Aminoschutzgruppe abspaltet und

ß) die Verbindung IV, worin $R^{11}$ Wasserstoff bedeutet, entweder als solche oder in Form eines reaktionsfähigen Derivates mit einer 2-syn-Oxyiminoessigsäure der allgemeinen Formel V,

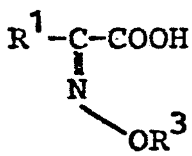

(V)

worin $R^1$ und $R^3$ die genannte Bedeutung besitzen, oder mit einem an der Carbonylgruppe aktivierten Derivat dieser Verbindung umsetzt und

α) eine gegebenenfalls vorhandene Schutzgruppe abspaltet und

ß) falls erwünscht, das erhaltene Produkt der allgemeinen Formel I in ein physiologisch verträgliches Säureadditionssalz überführt.

3. Verfahren gemäß Anspruch 2, dadurch gekennzeichnet, daß der nucleophile Austausch des Substituenten $R^{10}$ in Gegenwart von Neutralsalzionen, insbesondere von Jodid oder Thiocyanationen erfolgt.

4. Verfahren gemäß Anspruch 2, dadurch gekennzeichnet, daß der nucleophile Austausch des Substituenten $R^{10}$ in Gegenwart der dem Rest A zugrundeliegenden Base und von Tri-$C_1$-$C_4$-alkyljodsilan erfolgt.

5. Verfahren nach Anspruch 4, dadurch gekennzeichnet, daß das Tri-$C_1$-$C_4$-alkyljodsilan Trimethyl- oder Triethyljodsilan ist.

6. Gegen bakterielle Infektionen wirksame pharmazeutische Präparate, gekennzeichnet durch einen Gehalt an Cephemderivaten der allgemeinen Formel I.

7. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

8. Verwendung von Cephemderivaten der allgemeinen Formel I zur Bekämpfung bakterieller Infektionen.

PATENTANSPRÜCHE für Österreich:

1. Verfahren zur Herstellung von Cephemverbindungen der Formel I

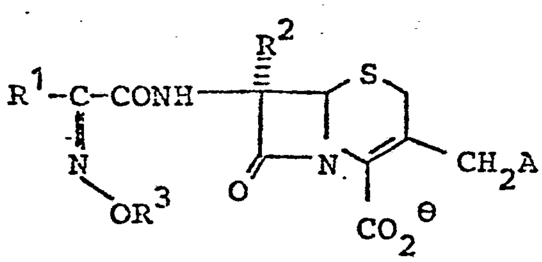

(I)

und deren physiologisch verträgliche Säureadditionssalze, worin bedeuten

$R^1$ einen 5- oder 6-gliedrigen heterocyclischen Ring, der durch folgende Formeln wiedergegeben wird

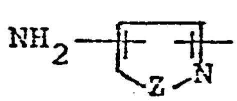 , 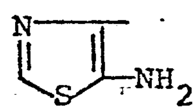 , 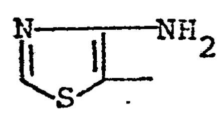 , 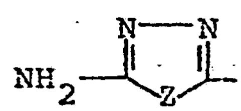 ,

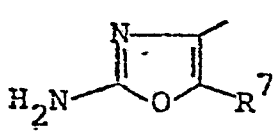 , 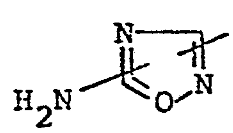 , 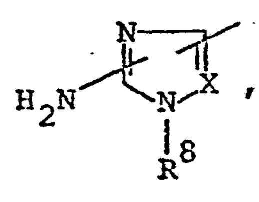 , 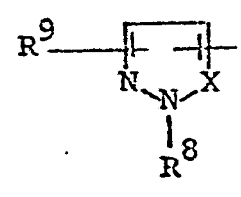 ,

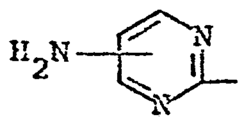 , 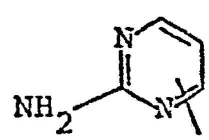 , 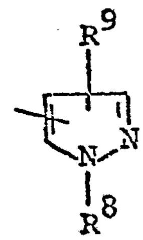 , 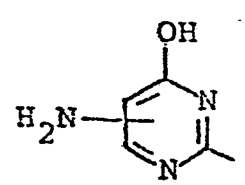 ,

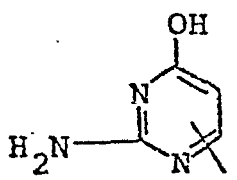 , 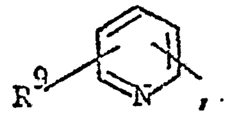 , 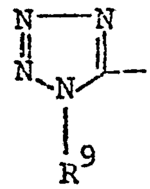 ,

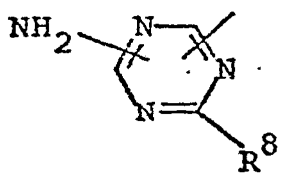 , 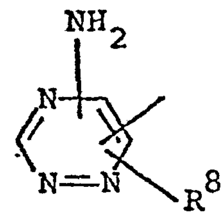

worin $R^7$ für Wasserstoff oder Halogen, $R^8$ für Wasserstoff oder einen gegebenenfalls substituierten $C_1$-$C_4$-Alkylrest, $R^9$ für Wasserstoff oder Amino, X für N oder CH, Z für O, S oder $NR^8$ steht,

$R^2$ Wasserstoff oder Methoxy

$R^3$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl, die Gruppe $-(CH_2)_n-(\overset{R^4}{\underset{R^5}{C}})_m-R^6$ , worin m und n jeweils für 0 oder 1 steht und

$R^4$ und $R^5$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$-Alkyl- und die $C_3$-$C_7$-Cycloalkylidengruppe noch weiter ein- oder mehrfach substituiert sein können,

$R^6$ eine COOH-; CN- oder $CONH_2$-Gruppe und

A eine gegebenenfalls substituierte Ammoniogruppe, die von einem tertiären aliphatischen oder cyclischen

Amin abgeleitet ist oder ein ungesättigtes, gegebenenfalls substituiertes 5-6-gliedriges heterocyclisches Kation der Formel 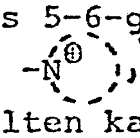, das weitere N, O oder S-Atome im Ring enthalten kann und an das mindestens ein zusätzlicher Ring ankondensiert sein kann und in der die $R^3$ O-Gruppe in syn-Position steht, dadurch gekennzeichnet, daß man

a) eine Verbindung der allgemeinen Formel II

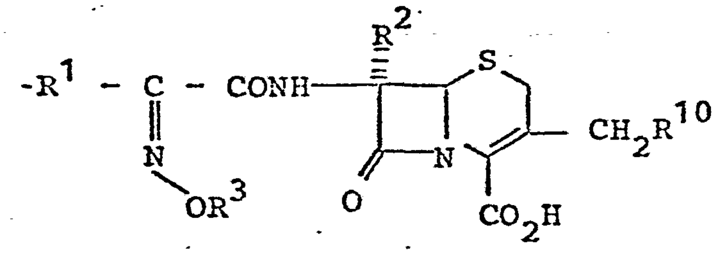

(II)

oder deren Salze, worin $R^1$, $R^2$ und $R^3$ die in Formel I genannte Bedeutung haben, und $R^{10}$ eine durch diejenige Base, die den Resten A der Formel I entspricht, austauschbare Gruppe bedeutet, mit dieser Base umsetzt und

α) eine gegebenenfalls vorhandene Schutzgruppe abspaltet und

ß) falls erwünscht, das erhaltene Produkt in ein physiologisch verträgliches Säureadditionssalz überführt,

oder

b) eine Verbindung der allgemeinen Formel III,

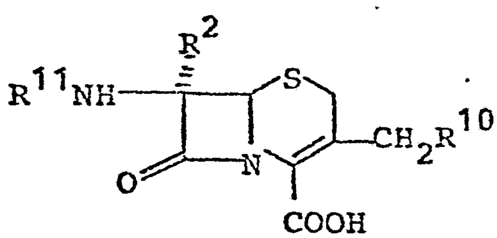

(III)

worin $R^2$ und $R^{10}$ die vorstehend für Formel II genannte Bedeutung haben und $R^{11}$ für Wasserstoff oder eine Aminoschutzgruppe steht, mit der Base, die dem in Formel I definierten Rest A zugrunde liegt, umsetzt unter Bildung der Verbindung der allgemeinen Formel IV,

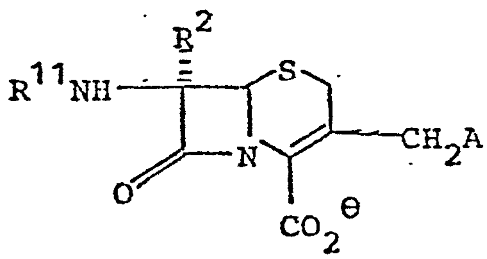

(IV),

in der $R^2$, $R^{11}$ und A die oben genannte Bedeutung haben und

α) eine gegebenenfalls vorhandene Aminoschutzgruppe abspaltet und

β) die Verbindung IV, worin $R^{11}$ Wasserstoff bedeutet, entweder als solche oder in Form eines reaktionsfähigen Derivates mit einer 2-syn-Oxyiminoessigsäure der allgemeinen Formel V,

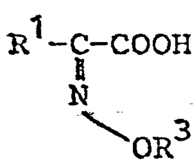

(V).

worin $R^1$ und $R^3$ die genannte Bedeutung besitzen, oder mit einem an der Carbonylgruppe aktivierten Derivat dieser Verbindung umsetzt und

α) eine gegebenenfalls vorhandene Schutzgruppe abspaltet und

β) falls erwünscht, das erhaltene Produkt der allgemeinen Formel I in ein physiologisch verträgliches Säureadditionssalz überführt.

2. Verfahren gemäß Anspruch 1, dadurch gekennzeichnet, daß der nucleophile Austausch des Substituenten $R^{10}$ in Gegenwart von Neutralsalzionen, insbesondere von Jodid oder Thiocyanationen erfolgt.

3. Verfahren gemäß Anspruch 1, dadurch gekennzeichnet, daß der nucleophile Austausch des Substituenten $R^{10}$ in Gegenwart der dem Rest A zugrundeliegenden Base und von Tri-$C_1$-$C_4$-alkyljodsilan erfolgt.

4. Verfahren nach Anspruch 3, dadurch gekennzeichnet, daß das Tri-$C_1$-$C_4$-alkyljodsilan Trimethyl- oder Triethyljodsilan ist.

5. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.